# EUROPÄISCHE PATENTSCHRIFT

(11) **EP 0 590 415 B1**
(45) Veröffentlichungstag und Bekanntmachung des Hinweises auf die Patenterteilung: **04.06.1997**
(21) Anmeldenummer: 93114857.1
(22) Anmeldetag: 15.09.1993
(51) Int. Cl.: C07D 271/06, A61K 31/41, A01N 43/82

(54) **1,2,4-Oxadiazolderivate zur Bekämpfung von Endoparasiten**
1,2,4-Oxadiazole derivatives for controlling endoparasites
Dérivés de 1,2,4-oxadiazole pour la lutte contre les endoparasites

(30) Priorität: 28.09.1992 DE 4232418
(43) Veröffentlichungstag der Anmeldung: 06.04.1994
(73) Patentinhaber: BAYER AG, 51368 Leverkusen (DE)
(72) Erfinder: Jeschke, Peter, Dr., D-51373 Leverkusen (DE); Lindner, Werner, Dr., D-51067 Köln (DE); Harder, Achim, Dr., D-51067 Köln (DE); Mencke, Norbert, Dr., D-51381 Leverkusen (DE)

(56) Entgegenhaltungen:
- EP-A- 0 007 529
- EP-A- 0 008 356
- EP-A- 0 010 163
- EP-A- 0 092 706
- EP-A- 0 245 818
- EP-A- 0 337 151
- EP-A- 0 492 249
- DE-A- 1 620 574
- GB-A- 2 163 427
- US-A- 4 012 377
- CHEMICAL ABSTRACTS, vol. 110, no. 19, 8. Mai 1989, Columbus, Ohio, US; abstract no. 173224u, Seite 776 ;
- CHEMICAL ABSTRACTS, vol. 98, no. 23, 6. Juni 1983, Columbus, Ohio, US; abstract no. 198238y, Seite 657 ;
- Mehlhorn/Piekarsky,"Grundri der Parasitenkunde,3.Auflage,Gustav Fischer Verlag,Stuttgart,1989,Seiten 1-13
- Römpps Chemie-Lexikon,Auflage,Franckh'sche Verlagshandlung,Stuttgart,1979,Seiten 2994,2995

## Beschreibung

Die vorliegende Erfindung betrifft die Verwendung von substituierten 1,2,4-Oxadiazolderivaten zur Bekämpfung von Endoparasiten, neue substituierte 1,2,4-Oxadiazolderivate und Verfahren zu ihrer Herstellung.

Verschiedene substituierte 1,2,4-Oxadiazolderivate sind bereits als Verbindungen mit Verwendung zur Bekämpfung parasitärer Protozoen (vgl. EP 7 529, EP 8 356, EP-A 492 249), als Analgetika (GB 1 198 726) oder als Herbizide (JP 57 175-177) bekannt geworden. Es ist jedoch nichts über eine Verwendung dieser Verbindungen gegen Endoparasiten bekannt.

Die vorliegende Erfindung betrifft:
1. Die Verwendung von substituierten 1,2,4-Oxadiazolderivaten der allgemeinen Formel (I) 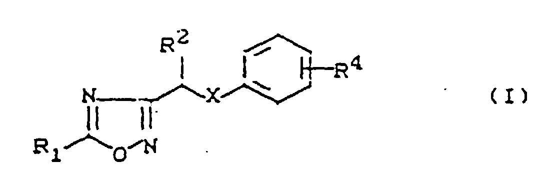 in welcher
   - R¹: für Wasserstoff, Alkyl das gegebenenfalls substituiert ist durch Halogen, Alkoxy, Hydroxy, Amino, Alkylamino, Dialkylamino, Cycloalkylamino sowie für Cycloalkyl, gegebenenfalls substituiertes Aryl oder Hetaryl steht,
   - R²: mit Wasserstoff und Alkyl steht,
   - X: für O, S, SO, SO₂ und N-R³ steht,
   - R³: für Wasserstoff, Alkyl, Alkylcarbonyl oder Halogenalkylcarbonyl steht,
   - R⁴: für gleiche oder verschiedene Reste aus der Reihe Wasserstoff, Halogen, Cyano, Nitro, Isothiocyanato, Amino, Aminoacyl, Alkylamino, Dialkylamino, Trialkylammonium-halogenid, Sulfonylamino, Hydroxy, Mercapto, Alkyl, Aralkyl, Halogenalkyl, Alkoxy, Aralkoxy, Halogenalkoxy, Aryl, Aryloxy, Arylthio, Arylsulfinyl, Arylsulfonyl, Alkylthio, Halogenalkylthio, Alkylsulfinyl, Halogenalkylsulfinyl, Alkylsulfonyl, Halogenalkylsulfonyl, Alkoxycarbonyl, Alkylcarbonyl, Alkylcarbonyloxy, Alkoxyalkyl, Alkoxyalkoxy, Hydroxyalkoxy, Hetarylmethylenoxy steht zur Herstellung von Mitteln gegen parasitäre Nematoden, Trematoden oder Cestoden.

   Die Verbindungen der Formel (I) sind teilweise bekannt und lassen sich analog zu bekannten Verfahren herstellen.
2. Die vorliegende Erfindung betrifft ferner neue substituierte 1,2,4-Oxadiazolderivate der allgemeinen Formel (I) 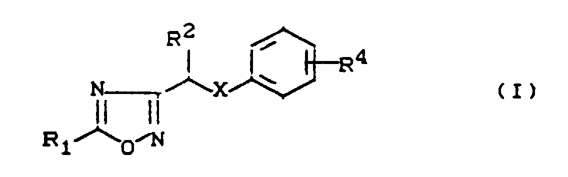 in welcher
   - R¹: für Wasserstoff, C₁-C₄-Alkyl das gegebenenfalls substituiert ist durch Halogen, gegebenenfalls substituiertes Aryl oder Hetaryl, insbesondere Furyl, Thienyl sowie Pyridyl steht,
   - R²: für Wasserstoff steht,
   - X: für S steht,
   - R⁴: für 2-Cl; 3-Cl; 2,3-Cl₂; 2,4-Cl₂; 2,5-Cl₂, 2,6-Cl₂, 2,4,6-Cl₃; 2-F, 3-Cl; 2,3-F₂; 3-Br; 3,4-OCHF-CF₂-O; 2-OCF₃; 4-OCF₃; 4-CF₃, 3,5-(CF₃)₂; 3,5-(CH₃)₂; 3,4-(OCH₃)₂; 2,5-(OCH₃)₂; 4-nC₉H₁₉; 4-N(C₂H₅)₂; 4-NH-CH₃; 4-NH-C₂H₅; 4-NH-CO-CH₃; 4-N⁺(CH₃)₃Cl⁻; 4-N⁺(CH₃)₃I⁻ steht,
   wobei R⁴ für den Fall, daß R¹ eine andere Bedeutung als Wasserstoff hat, zusätzlich für 3,4-Cl₂; 2,4,5-Cl₃; 4-F; 2-Br; 3-CF₃; 2-CH₃; 3-CH₃; 2,4-(CH₃)₂; 2,6(CH₃)₂; 4-OH; 4-O-OC-CH₃; 2-OCH₃; 3-OCH₃; 4-OCH₃; 4-tC₄H₉; 4-NCS; 4-NH₂; 4-NH-CH₃; 4-N(CH₃)₂; 4-N(C₂H₅)₂ steht,
   sowie die Verbindung der Formel (IA) 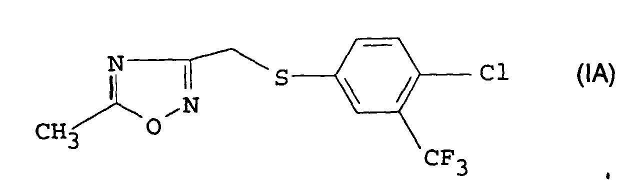 Verfahren zur Herstellung der neuen substituierten 1,2,4-Oxadiazolderivate der Formel (I) und (IA) gemäß (2) oben 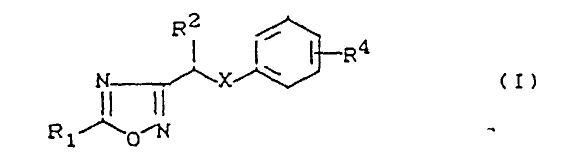
in welcher
X, R¹, R², R³ und R⁴ die in Anspruch 3 angegebene Bedeutung besitzen,
dadurch gekennzeichet, daß man
a) Verbindungen der Formel (II) 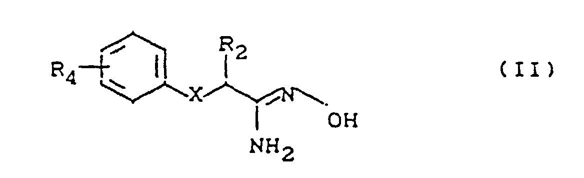 in welcher
   X, R², R³ und R⁴ die oben angegebene Bedeutung besitzen,
   mit Carbonsäurederivaten der Formeln (III), (IV) oder (V)

      R¹-C(O-R)₃ (III)

      (R¹-CO)₂O (IV)

      R¹-CO-Y (V)

      worin
      R¹ die oben angegebene Bedeutung hat und R für Methyl oder Ethyl steht, Y für eine geeignete Abgangsgruppe steht, umsetzt oder
   b) Verbindungen der Formel (VI) 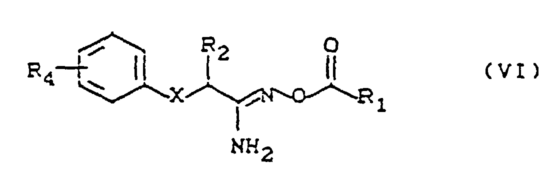 in welcher
      X, R¹, R², R³ und R⁴ die oben angegebene Bedeutung besitzen,
      gegebenenfalls in Gegenwart eines Verdünnungsmittels und gegebenenfalls in Gegenwart eines Reaktionshilfsmittels cyclisiert, oder
   c) Verbindungen der Formel (VII) 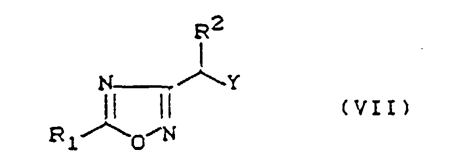 in welcher
      R¹ und R² die oben angegebene Bedeutung haben,
      Y für eine geeignete Abgangsgruppe steht,
         mit Verbindungen der Formel (VIII) 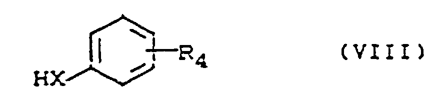
      in welcher
      X und R⁴ die oben angegebene Bedeutung besitzen,
      in Gegenwart eines Verdünnungsmittels und gegebenenfalls in Gegenwart eines Reaktionshilfsmittels umsetzt.

Die erfindungsgemäßen substituierten 1,2,4-Oxadiazol-derivate sind durch die Formel (I) allgemein definiert.

Bevorzugt sind Verbindungen der Formel (I), in welcher
- R¹: für Wasserstoff, C₁-C₄-Alkyl das gegebenenfalls substituiert ist durch Halogen, insbesondere Fluor, Chlor oder Brom, C₁-C₄-Alkoxy, insbesondere Methoxy oder Ethoxy, Hydroxy, Amino, C₁-C₄-Alkylamino, insbesondere Methylamino, C₁-C₄-Dialkylamino, insbesondere Dimethylamino oder Diethylamino sowie Cyclo-C₃-C₇-alkylamino, insbesondere Cyclopropylamino, Cyclopentylamino oder Cyclohexylamino, Cyclo-C₃-C₇-alkyl, insbesondere Cyclopropyl, Cyclopentyl oder Cyclohexyl, gegebenenfalls substituiertes Phenyl oder Hetaryl, insbesondere Furyl, Thienyl sowie Pyridyl steht,
- R²: für Wasserstoff und C₁-C₄-Alkyl, insbesondere Methyl steht,
- X: für O, S, SO, SO₂ und -N-R³ steht,
- R³: für Wasserstoff, C₁-C₄-Alkyl, insbesondere Methyl oder Ethyl, C₁-C₄-Alkylcarbonyl, insbesondere Acetyl oder Propionyl, Halogen-C₁-C₄-alkylcarbonyl, insbesondere Trifluoracetyl steht,
- R⁴: für gleiche oder verschiedene Reste aus der Reihe Wasserstoff, Halogen, insbesondere Fluor, Chlor oder Brom, Cyano, Nitro, Isothiocyanato, Amino, C₁-C₄-Alkylamino, insbesondere Methylamino oder Ethylamino, C₁-C₄-Dialkylamino, insbesondere Dimethylamino, C₁-C₄-Trialkylammonium-halogenid, insbesondere Trimethylammonium-bromid, -chlorid oder -iodid, Sulfonylamino, Hydroxy, Mercapto, C₁-C₉-Alkyl, insbesondere Methyl, Ethyl, Isopropyl, Butyl, Hexyl, Oktyl oder Nonyl, Aralkyl, Halogen-C₁-C₄-alkyl, insbesondere Trifluormethyl, C₁-C₄-Alkoxy, insbesondere Methoxy, Ethoxy, Methylendioxy oder Ethylendioxy, die gegebenenfalls durch Fluor oder Chlor substituiert sind, Halogen-C₁-C₄-alkoxy, insbesondere Trifluormethoxy, Fluorchlorethoxy, Aralkoxy, Aryl, Aryloxy, Arylthio, Arylsulfinyl, Arylsulfonyl, C₁-C₄-Alkylthio, insbesondere Methylthio, C₁-C₄-Halogenalkylthio, insbesondere Trifluormethylthio, Fluorchlormethylthio, C₁-C₄-Alkylsulfinyl, insbesondere Methylsulfinyl, C₁-C₄-Halogenalkylsulfinyl, insbesondere, Trifluormethylsulfinyl, C₁-C₄-Alkylsulfonyl, insbesondere Methylsulfonyl, C₁-C₄-Halogenalkylsulfonyl, insbesondere Trifluormethylsulfonyl, Alkoxycarbonyl, insbesondere Methoxy- oder Ethoxycarbonyl, Alkylcarbonyl, insbesondere Acetyl oder Propionyl sowie Oxadiazolylmethylenoxy das durch einen der bei R¹ angegebenen Reste substituiert ist, steht.

Besonders bevorzugt sind Verbindungen der Formel (I) 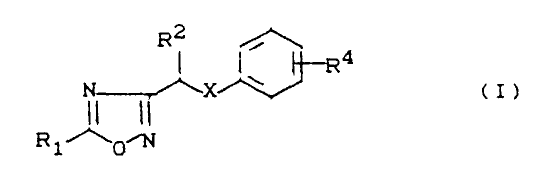 in welcher
- R¹: für Wasserstoff, C₁-C₄-Alkyl, insbesondere Methyl, Ethyl, Isopropyl oder sec. Butyl, C₁-C₄-Halogenalkyl, insbesondere Trichlormethyl oder Trifluormethyl steht,
- R²: für Wasserstoff, C₁-C₄-Alkyl, insbesondere Methyl steht,
- X: für O, S, SO, SO₂ und -N-R³ steht,
- R³: für Wasserstoff, C₁-C₄-Alkylcarbonyl, insbesondere Acetyl oder Propionyl, Halogen-C₁-C₄-alkylcarbonyl, insbesondere Trifluoracetyl steht,
- R⁴: für gleiche oder verschiedene Reste aus der Reihe Wasserstoff, Halogen, insbesondere Fluor, Chlor oder Brom, Nitro, Isothiocyanato, Amino, C₁-C₄-Alkylamino, insbesondere Methylamino oder Ethylamino, C₁-C₄-Dialkylamino, insbesondere Dimethylamino, C₁-C₄-Trialkylammonium-halogenid, insbesondere Trimethylammonium-bromid, -chlorid oder -iodid, Hydroxy, Mercapto, C₁-C₉-Alkyl, insbesondere Methyl, Ethyl, Isopropyl, Butyl, Hexyl, Oktyl oder Nonyl, Halogen-C₁-C₄-alkyl, insbesondere Trifluormethyl, C₁-C₄-Alkoxy, insbesondere Methoxy, Ethoxy, Methylendioxy oder Ethylendioxy, die gegebenenfalls durch Fluor oder Chlor substituiert sind, Halogen-C₁-C₄-alkoxy, insbesondere Trifluormethoxy, chlorethoxy, C₁-C₄-Alkylthio, insbesondere Methylthio, C₁-C₄-Halogenalkylthio, insbesondere Trifluormethylthio, Fluorchlormethylthio, C₁-C₄-Alkylsulfinyl, insbesondere Methylsulfinyl, C₁-C₄-Halogenalkylsulfinyl, insbesondere Trifluormethylsulfinyl, C₁-C₄-Alkylsulfonyl, insbesondere Methylsulfonyl, C₁-C₄-Halogenalkylsulfonyl, insbesondere Trifluormethylsulfonyl sowie für 3-(1,2,4-Oxadiazolyl)-methylenoxy, das durch einen der bei R¹ angegebenen Reste in 5-Stellung substituiert ist, steht.

Ganz besonders bevorzugt sind Verbindungen der Formel (I)
in welcher
- R¹: für Wasserstoff, C₁-C₄-Alkyl, insbesondere Methyl, C₁-C₄-Halogenalkyl, insbesondere Trichlormethyl oder Trifluormethyl steht,
- R²: für Wasserstoff, C₁-C₄-Alkyl, insbesondere Methyl steht,
- X: für O, S, SO, SO₂ und -N-R³ steht,
- R³: für Wasserstoff, C₁-C₄-Alkylcarbonyl, insbesondere Acetyl, Halogen-C₁-C₄-alkylcarbonyl, insbesondere Trifluoracetyl steht,
- R⁴: für gleiche oder verschiedene Reste aus der Reihe Wasserstoff, Halogen, insbesondere Fluor, Chlor oder Brom, Nitro, Amino, C₁-C₄-Dialkylamino, insbesondere Dimethylamino, C₁-C₄-Trialkylammonium-halogenid, insbesondere Trimethylammonium-bromid, - chlorid oder -iodid, Hydroxy, C₁-C₄-Alkyl, insbesondere Methyl, Ethyl oder Isopropyl, Halogen-C₁-C₄-alkyl, insbesondere Trifluormethyl, C₁-C₄-Alkoxy, insbesondere Methoxy, Methylendioxy oder Ethylendioxy, die gegebenenfalls durch Fluor oder Chlor substituiert sind, Halogen-C₁-C₄-alkoxy, insbesondere Trifluormethoxy, Fluorchlorethoxy, C₁-C₄-Alkylthio, insbesondere Methylthio, C₁-C₄-Halogenalkylthio, insbesondere Trifluormethylthio, Fluorchlormethylthio sowie für 3-(5-Trifluormethyl-1,2,4-oxadiazolyl)-methylenoxy steht.

Die erfindungsgemäß zu verwendenden Verbindungen der Formel (I), in denen R² vorzugsweise für Methyl steht, enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Im einzelnen seien folgende Verbindungen der Formel (I) genannt, in welcher die Reste R¹, R², R³ und R⁴ die folgende Bedeutung haben: 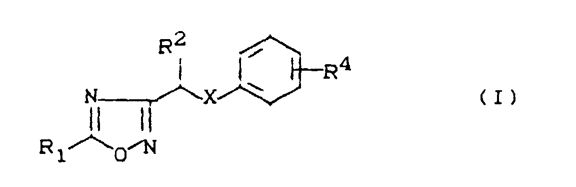
3-(2-Fluor-4-chlor-phenoxymethyl)-1,2,4-oxadiazol,
3-(2-Fluor-3-chlor-phenoxymethyl)-5-methyl-1,2,4-oxadiazol,
3-(2,6-Dichlor-phenoxymethyl)-1,2,4-oxadiazol,
3-(2,6-Dichlor-phenoxymethyl)-5-methyl-1,2,4-oxadiazol,
3-(3,4-Dimethoxy-phenylthiomethyl)-1,2,4-oxadiazol,
3-(2,5-Dimethoxy-phenylthiomethyl)-5-methyl-1,2,4-oxadiazol,
3-(2,6-Dichlor-phenylsulfoxylmethyl)-5-methyl-1,2,4-oxadiazol,
3-(2,6-Dimethyl-phenylsulfoxylmethyl)-5-methyl-1,2,4-oxadiazol,
3-(2,6-Dichlor-phenylaminomethyl)-1,2,4-oxadiazol,
3-(2,6-Dichlor-phenylaminomethyl)-5-methyl-1,2,4-oxadiazol,
3-(2-Fluor-3-chlor-phenylaminomethyl)-1,2,4-oxadiazol,
3-(2-Fluor-3-chlor-phenylaminomethyl)-5-methyl-1,2,4-oxadiazol,
3-(4-Methoxy-phenylaminomethyl)-5-methyl-1,2,4-oxadiazol,
3-(N-Trifluoracetyl-2,6-dichlor-phenylaminomethyl)-5-methyl-1,2,4-oxadiazol,
3-(N-Acetyl-2,6-dichlor-phenylaminomethyl)-5-methyl-1,2,4-oxadiazol.

Von den neuen Verbindungen der Formel (I) sind diejenigen bevorzugt und besonders bevorzugt, in denen die Substituenten die oben angegebenen bevorzugten Definitionen besitzen.

Die Verbindungen der Formel (I) sind teilweise bekannt, sie lassen sich nach den oben unter Punkt 3 angegebenen Verfahren a) bis c) herstellen (vgl. z.B. EP 8 356, EP 7 529; vgl. auch Reviews: F. Eloy Fortschr. chem. Forsch. 4 (1965) S. 807; L.C. Behr in "The Chemistry of Heterocyclic Compounds", Vol. 17, (1962) S. 245; B.C. Leallyn in "Advances in Heterocyclic Chemistry", Vol. 20, Edited by A.R. Katritzky, A.J. Boulton, Adademic Press ^{·} New York ^{·} San Francisco ^{·} London, 1976, S. 65).

Setzt man bei Verfahren 3a) zur Herstellung der neuen substituierten 1,2,4-Oxadiazolderivate als Verbindungen der Formel (II) 3-Chlor-2-methyl-phenoxy-acetamidoxim und als Verbindung der Formel (III) Orthoameisensäuretriethylester ein, so läßt sich das Verfahren durch folgendes Reaktionsschema wiedergeben.

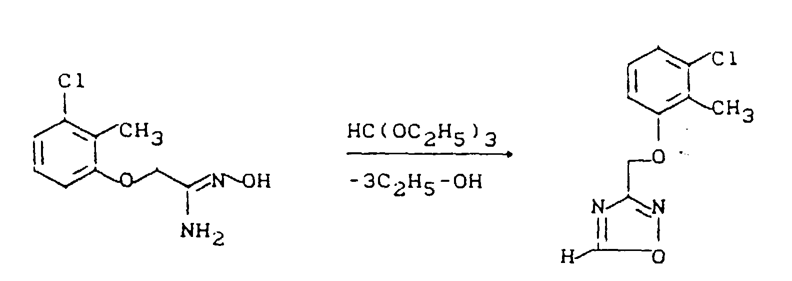

Die zur Durchführung des erfindungsgemäßen Verfahrens 3a) als Ausgangsstoffe benötigten Amidoxime sind durch die Formel (II) allgemein definiert. In dieser Formel stehen X, R², R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Verbindungen der Formel (II) sind teilweise bekannt (vgl. z.B. Cervena' et. al. Collect. Czech. Chem. Commun. 46 (1981) 5, S. 1188-1198, EP 8 356, EP 7 529) bzw. können nach den dort beschriebenen Verfahren erhalten werden.

Die außerdem für die Durchführung des erfindungsgemäßen Verfahrens 3a) als Ausgangsstoffe zu verwendenden Carbonsäureorthoester sind allgemein durch die Formel (III) definiert. Die darüber hinaus für die Durchführung des erfindungsgemäßen Verfahrens 3a) in Frage kommenden Carbonsäurederivate sind durch die Formeln (IV) und (V) allgemein definiert. In den Formeln (III) bis (V) hat R¹ die Bedeutung die bereits im Zusammenhang mit der Beschreibung der erfindungsgemäßen Stoffe der Formel (I) als bevorzugt für diesen Substituenten genannt wurden. In der Formel (IV) hat Y die Bedeutung einer geeigneten Abgangsgruppe wie beispielsweise Halogen oder Alkoxy. Die Carbonsäureorthoester der Formel (III) sowie die Carbonsäurederivate der Formeln (IV) und (V) sind allgemein bekannte Verbindungen der organischen Chemie.

Im einzelnen seien folgende Verbindungen der Formel (II) genannt:

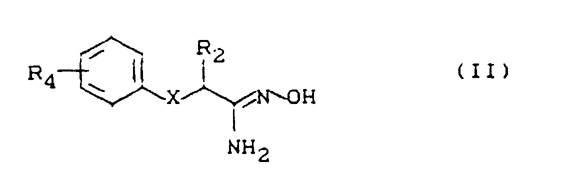

| X | R² | R⁴ | X | R² | R⁴ |
|---|---|---|---|---|---|
| O | H | 4-OCH₃ | S | CH₃ | 2-F, 3-Cl |
| O | CH₃ | 4-OCH₃ | S | CH₃ | 2,6-Cl₂ |
| O | H | 2-F, 3-Cl | S | CH₃ | 2,6-(CH₃)₂ |
| O | CH₃ | 2-F, 3-Cl | S | CH₃ | 2-OCH₂ |
| O | H | 2,6-Cl₂ | S | CH₃ | 3-OCH₃ |
| O | CH₃ | 2,6-Cl₂ | NH | CH₃ | 2-F, 3-Cl |
| O | H | 2,6-(CH₃)₂ | NH | H | 2,6-Cl₂ |
| O | H | 2-CH₃, 3-Cl | NH | H | 2,6-(CH₃)₂ |
| O | H | 3-OCH₃ | NH | CH₃ | 2-F, 3-Cl |

Die Verbindungen der Formel (II) und (III) werden vorzugsweise in Gegenwart eines sauren Katalysators umgesetzt. Als solche kommen dabei praktisch alle Mineralsäuren oder Lewis-Säuren in Frage. Zu den Mineralsäuren gehören vorzugsweise Halogenwasserstoffsäuren wie Fluorwasserstoffsäure, wasserstoffsäure, Bromwasserstoffsäure oder Iodwasserstoffsäure sowie Schwefelsäure, Phosphorsäure, Phosphorige Säure, Salpetersäure und zu den Lewis-Säuren gehören vorzugsweise Aluminium(III)-chlorid, Bortrifluorid oder sein Etherat, Titan(IV)-chlorid, Zinn(IV)-chlorid.

Besonders bevorzugt werden folgende Lewis-Säuren eingesetzt:

Bortrifluorid oder sein Etherat, Aluminium(III)-chlorid.

Das Verfahren 3a) wird durchgeführt, indem man Verbindungen der Formel (II) mit einem Überschuß an Verbindungen der Formel (III) zusammengibt und in Gegenwart eines sauren Katalysators erhitzt. Hierbei ist die Verbindung (II) zugleich Verdünnungsmittel. Die Reaktionsdauer beträgt ca. 1 bis 4 Stunden. Die Reaktion wird bei Temperaturen zwischen +20°C und +200°C, bevorzugt zwischen +100°C und +155°C durchgeführt. Vorzugsweise arbeitet man bei dem Druck, der sich beim Erhitzen auf die erforderliche Reaktionstemperatur unter den Reaktionsbedingungen einstellt.

Nach vollendeter Umsetzung wird das Reaktionsgemisch abgekühlt, im Vakuum eingeengt, der verbleibende Rückstand in einem organischen Lösungsmittel aufgenommen und in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Alternativ kann diese Umsetzung auch mit einem Meerwein-Reagenz (z.B. einem Dialkylacetal von Dimethylformamid) oder dem Vilsmeier-Haack-Reagenz (POCl₃, N,N-Dimethylformamid) durchgeführt werden.

Setzt man bei Verfahren 3b) als Verbindungen der Formel (VI) O-Benzoyl-(4-chlor-phenoxy)-acetamidoxim ein, läßt sich das Verfahren durch folgendes Reaktionsschema beschreiben:

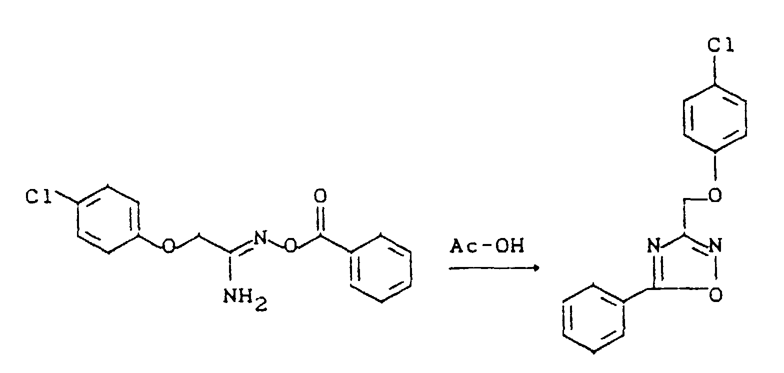

Bevorzugt werden bei Verfahren 3b) die Verbindungen der Formel (VI) eingesetzt, bei denen X und die Reste R¹, R², R³ und R⁴ die bei den Verbindungen der Formel (I) genannten bevorzugten und besonders bevorzugten Bedeutungen besitzen.

Die Verbindungen der Formel (VI) können sowohl in situ aus Verbindungen der Formel (II) und geeigneten Carbonsäurederivaten der Formel (IV) und (V) bei Verfahren 3a) entstehen aber auch wie hier bei Verfahren 3b) isoliert eingesetzt werden.

Die Cyclisierung der Verbindungen der Formel (VI) wird vorzugsweise unter Verwendung von Verdünnungsmitteln und gegebenenfalls in Gegenwart eines Reaktionshilfsmittels durchgeführt.

Als Verdünnungsmittel zur Durchführung des erfindungsgemäßen Verfahrens 3b) kommen alle inerten organischen Lösungsmittel in Frage.

Als Beispiele sind zu nennen: Halogenkohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Di-n-butylether, Di-isobutylether, Di-iso-amylether, Di-isopropylether, Anisol, Phenetol, Cyclohexylmethylether, Diethylether, Ethylenglycoldimethylether, Tetrahydrofuran, Dioxan, Dichlordiethylether, Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylethylketon, Carbonsäuren wie Essigsäure, Propionsäure, Buttersäure. Auch Gemische der genannten Lösungs- und Verdünnungsmittel kommen in Betracht.

Bevorzugt sind Carbonsäuren wie Essigsäure oder aromatische Kohlenwasserstoffe wie Toluol und Xylol.

Als Reaktionshilfsmittel können alle geeigneten Dehydratisierungsreagenzien Verwendung finden, wie beispielsweise Dicyclohexylcarbodiimid [DCC] (vgl. z.B. F. Eloy Fortschr. chem. Forsch. 4 (1965) S. 807).

Das Verfahren 3b) wird durchgeführt, indem man Verbindungen der Formel (VI) in einem geeigneten Verdünnungsmittel, gegebenenfalls in Gegenwart eines geeigneten Reaktionshilfsmittel erhitzt. Die Reaktionsdauer beträgt ca. 1 bis 10 Stunden. Die Reaktion wird bei Temperaturen zwischen +20°C und +200°C, bevorzugt zwischen +70°C und +170°C durchgeführt. Vorzugsweise arbeitet man bei dem Druck, der sich beim Erhitzen auf die erforderliche Reaktionstemperatur unter den Reaktionsbedingungen einstellt.

Nach vollendeter Umsetzung wird das Reaktionsgemisch abgekühlt, der gesamte Reaktionsansatz eingeengt, in einem organischen Lösungsmittel aufgenommen und in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 3c) zur Herstellung der neuen 1,2,4-Oxadiazolderivate als Verbindungen der Formel (VII) 3-Chlormethyl-5-methyl-1,2,4-oxadiazol und als Verbindungen der Formel (VIII) 2,3-Dichlor-thiophenol ein, so läßt sich das Verfahren durch folgendes Reaktionsschema wiedergeben:

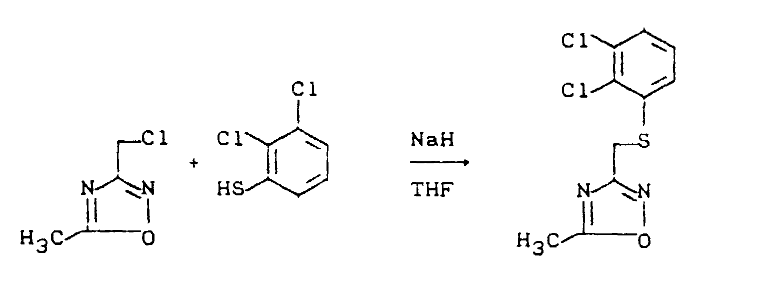

Die zur Durchführung des erfindungsgemäßen Verfahrens 3c) als Ausgangsstoffe benötigten 3,5-disubstituierten 1,2,4-Oxadiazole sind durch die Formel (VII) allgemein definiert. In dieser Formel stehen Y, R¹ und R² vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Verbindungen der Formel (VII) sind teilweise bekannt (vgl. z.B. DE-OS 2 406 786, GB 2 205 101; G. Palazzo J. Heterocyclic Chem. 16 (1979) S. 1469) bzw. können nach den dort beschriebenen Verfahren erhalten werden.

Im einzelnen seien folgende Verbindungen der Formel (VII) als Startkomponente genannt, in welcher Y sowie die Reste R¹ und R² die folgende Bedeutung haben.
3-Chlormethyl-1,2,4-oxadiazol,
3-Chlormethyl-5-methyl-1,2,4-oxadiazol,
3-Chlormethyl-5-trifluormethyl-1,2,4-oxadiazol,
3-Chlormethyl-5-trichlormethyl-1,2,4-oxadiazol,
3-Chlormethyl-5-ethyl-1,2,4-oxadiazol,
3-Chlormethyl-5-(2-chlor-6-fluor-phenyl)-1,2,4-oxadiazol.

Die Reaktion der Verbindungen der Formel (VII) wird vorzugsweise unter Verwendung von Verdünnungsmitteln und in Gegenwart eines basischen Reaktionshilfsmittels durchgeführt.

Als basische Reaktionshilfsmittel können alle geeigneten Säurebindemittel eingesetzt werden wie Amine, insbesondere tertiäre Amine sowie Alkali- und Erdalkaliverbindungen.

Beispielhaft seien dafür erwähnt die Hydride, Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methylpiperidin, N-Methyl-imidazol, N-Methyl-pyrrol, N-Methyl-morpholin, N-Methyl-hexamethylenimin, Pyridin, Chinolin, α-Picolin, β-Picolin, Isochinolin, Pyrimidin, Acridin, N,N,N',N'-Tetra-methylendiamin, N,N,N'N'-Tetra-ethylendiamin, Chinoxalin, N-Propyl-diisopropylamin, N,N'-Dimethylcyclohexylamin, 2,6-Lutidin, 2,4-Lutidin, Triethylendiamin, Diazabicyclooctan (DABCO), Diazabicyclononen (DBN) oder Diazabicycloundecen (DBU).

Vorzugsweise verwendet man tertiäre Amine, wie beispielsweise Triethylamin, N-Methyl-morpholin oder Heteroaromaten bzw. Hydride oder Hydroxide des Kaliums oder Natriums.

Das Verfahren 3c) wird durchgeführt, indem man Verbindungen der Formel (VII) mit einem geringen Überschuß an Verbindungen der Formel (VIII) zusammengibt und in Gegenwart eines basischen Reaktionshilfsmittels erhitzt. Die Reaktionsdauer beträgt ca. 5 bis 30 Stunden. Die Reaktion wird bei Temperaturen zwischen +20°C und +200°C, bevorzugt zwischen +70°C und +170°C durchgeführt. Vorzugsweise arbeitet man bei dem Druck, der sich beim Erhitzen auf die erforderliche Reaktionstemperatur unter den Reaktionsbedingungen einstellt.

Nach vollendeter Umsetzung wird das Reaktionsgemisch abgekühlt, der gesamte Reaktionsansatz filtriert und im Vakuum eingeengt, das zurückbleibende Rohprodukt wird in an sich bekannter Weise aufgearbeitet. Die anfallenden Produkte lassen sich in üblicher Weise durch Umkristallisieren, Vakuumdestillation oder bevorzugt durch Säulenchromatographie reinigen (vgl. auch die Herstellungsbeispiele).

Bei den nach Verfahren 3a) bis 3c) dargestellten Verbindungen der allgemeinen Formel (I) mit einem Arylthiomethyl-Rest, kann die schwefeltragende Gruppe oxidiert werden. Die Oxidation kann nach üblichen Verfahren mit geeigneten Oxidationsmitteln, wie Peroxide (z.B. H₂O₂), Permanganat, Perbenzoesäure, oder mit einem Gemisch aus Kaliumperoxomonosulfat, 2KHSO₅, KHSO₄, und einem Lösungsmittel oder Lösungsmittelgemisch (z.B. Wasser, Essigsäure, Methanol) oxidiert werden (vgl. A.R. Katritzky, C.W. Rees in Comprehensive Heterocyclic Chemistry, Pergamon Press, Oxford, New York, 1984, Vol. 3, S. 96; D.J. Brown et al. Chem. Soc. (C), 1971, S. 256).

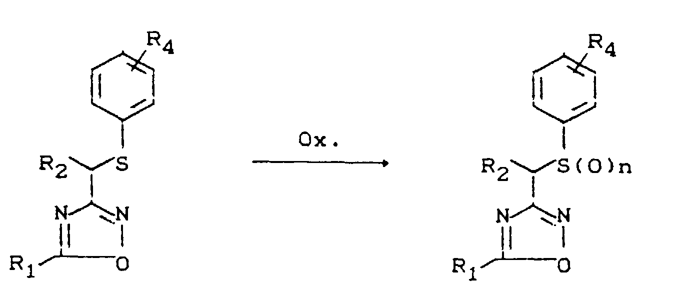

Die Oxidation kann auch mittels Katalysatoren gestartet oder beschleunigt werden.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.; Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.; Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.; Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 14 | 10 |
| 18 | 10 |
| 19 | 10 |
| 21 | 10 |
| 28 | 10 |
| 33 | 10 |
| 35 | 10 |
| 36 | 10 |
| 38 | 10 |
| 45 | 10 |
| 49 | 10 |
| 52 | 10 |

### Beispiel B

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 1 | 10 |
| 13 | 10 |
| 15 | 10 |
| 16 | 10 |
| 17 | 10 |
| 45 | 10 |

### Herstellungsbeispiel

### Beispiel 1

### 3-(3-Chlor-2-methyl-phenoxymethyl)-1,2,4-oxadiazol

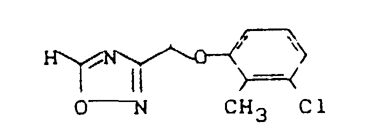

7,0 g (32,6 mMol) 3-Chlor-2-methyl-phenoxy-acetamidoxim werden in 70 ml Orthoameisensäuretriethylester vorgelegt und bei Raumtemperatur 5 Tropfen Bortrifluorid-etherat zugegeben. Dann wird bis zum vollständigen Umsatz (ca. 1 Stunde) bei Rückflußtemperatur gerührt und der gesamte Ansatz im Vakuum eingeengt. Der Rückstand wird in 100 ml Methylenchlorid aufgenommen und nacheinander mit 100 ml 2N-Salzsäure, gesättigter Natriumcarbonatlösung und Wasser gewaschen. Die organische Phase wird über Natriumsulfat getrocknet und anschließend das Lösungsmittel abdestilliert. Der zurückbleibende ölige Rückstand wird im Kugelrohr destilliert. Man erhält 4,2 g (57,4 % der Theorie) 3-(3-Chlor-2-methyl-phenoxymethyl)-1,2,4-oxadiazol.
Kp_{0,1 mbar}: 130°C
¹H-NMR (DMSO-d₆,δ,ppm) 2,22 (s, 3H, arom.-CH₃); 5,40 (s, 2H, -CH₂-O-); 6,86 - 7,30 (m, 3H, arom.-H); 9,69 (s, 1H, hetarom.-H).

### Beispiel 2

### 3-(3-Chlor-2-methyl-phenoxymethyl)-5-methyl-1,2,4-oxadiazol

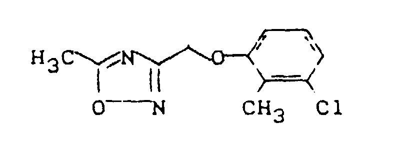

7,0 g (32,6 mMol) 3-Chlor-2-methyl-phenoxy-acetamidoxim, 14,0 g (142,8 mMol) Natriumacetat werden in 80 ml Diglyme vorgelegt und mit 8,6 g (84,2 mMol) Essigsäureanhydrid versetzt. Das Reaktionsgemisch wird bis zum vollständigen Umsatz (ca. 2 Stunden) bei Rückflußtemperatur gerührt. Anschließend wird der gesamte Reaktionsansatz eingeengt, mit Wasser versetzt und mit Essigsäureethylester extrahiert. Nach dem Abdestillieren des Lösungsmittels wird das zurückbleibende Rohprodukt über eine Kieselgelsäule (Kieselgel 60-Merck, Korngröße; 0,040 bis 0,063 mm) mit dem Fließmittel Methylenchlorid chromatographiert. Man erhält 4,4 g (56,5 % der Theorie) 3-(3-Chlor-2-methyl-phenoxymethyl)-5-methyl-1,2,4-oxadiazol.
Fp: 69°C
¹H-NMR (CDCl₃,δ,ppm) 2,30 (s, 3H, arom.-CH₃); 2,61 (s, 3H, hetarom.-CH₃); 5,14 (s, 2H, -CH₂-O-); 6,84 - 7,10 (m, 3H, arom.-H);

### Beispiel 3

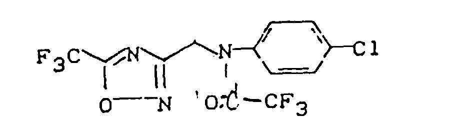

8,0 g (40,0 mMol) 4-Chlor-phenylamino-acetamidoxim werden in 90 ml absolutem Tetrahydrofuran vorgelegt, mit 12,75 g (60,7 mMol) Trifluoressigsäureanhydrid versetzt und 1,5 Stunden bei Raumtemperatur gerührt. Danach wird der gesamte Reaktionsansatz im Vakuum eingeengt und das zurückbleibende Rohprodukt über eine Kieselgelsäule (Kieselgel 60-Merck, Korngröße 0,040 bis 0,063 mm) mit dem Fließmittel Chloroform : Essigsäureethylester (10:1) chromatographiert. Man erhält 5,4 g (36,1 % der Theorie) 3-(N-Trifluoracetyl-4-chlor-phenylaminomethyl)-5-trifluormethyl-1,2,4-oxadiazol und 3,0 g (27,0 %) 3-(4-Chlor-phenylaminomethyl)-5-trifluormethyl-1,2,4-oxadiazol.

### 3-(N-Trifluoracetyl-4-chlor-phenylaminomethyl)-5-trifluormethyl-1,2,4-oxadiazol

¹H-NMR (CDCl₃,δ,ppm): 5,08 (s, 2H, -CH₂-N-); 7,36; 7,43 (2d, 4H, arom.-H; J = 8,8 Hz)

### Beispiel 4

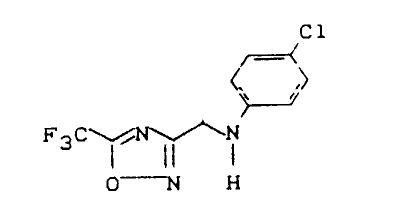

### 3-(4-Chlor-phenylaminomethyl)-5-trifluormethyl-1,2,4-oxadiazol

¹H-NMR (CDCl₃,δ,ppm): 4,34 (br.s, 1H, -NH); 4,55 (s, 2H, -CH₂-O-); 6,63; 7,15 (2d, 4H, arom.-H; J = 8,8 Hz)

### Beispiel 5

### 3-Methylenoxy-[2,3,5-trimethyl-4-(3-methylenoxy-5-trifluormethyl-1,2,4-oxadiazolyl)]phenyl-5-trifluormethyl-1,2,4-oxadiazol

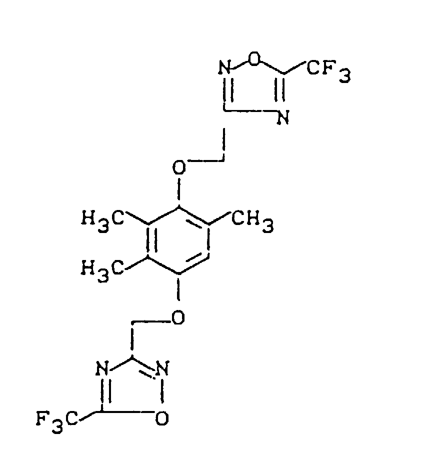

3,8 g (12,8 mMol) Bis-amidoxim werden in 40 ml absolutem Tetrahydrofuran vorgelegt und bei Raumtemperatur mit 8,5 g (40,4 mMol) Trifluoressigsäureanhydrid versetzt. Nach einstündigem Rühren bei Raumtemperatur wird der gesamte Reaktionsansatz im Vakuum eingeengt und das zurückbleibende Rohprodukt über eine Kieselgelsäule (Kieselgel 60-Merck, Korngröße: 0,040 bis 0,063 mm) mit dem Fließmittel Methylenchlorid chromatographiert. Man erhält 2,6 g (44,2 % der Threorie) 3-Methylenoxy-[2,3,5-trimethyl-4-(3-methylenoxy-5-trifluormethyl-1,2,4-oxadiazolyl)] phenyl-5-trifluormethyl-1,2,4-oxadiazol.
Fp.: 64°C
¹H-NMR (CDCl₃),δ.ppm): 2,09; 2,23 (3s, 3H, -CH₃); 4,87; 5,15 (2s, 4H, -CH₂-O); 6,59 (s, 1H, arom.-H)

### Beispiel 6

### 3-(4-Chlor-phenoxymethyl)-5-phenyl-1,2,4-oxadiazol

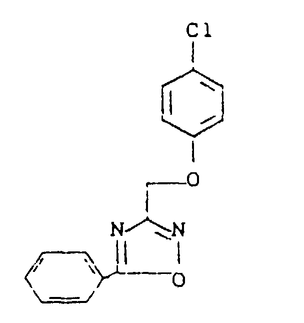

4,4 g (14,4 mMol) O-Benzoyl-(4-chlor-phenoxy)-acetamidoxim werden bis zur vollständigen Cyclisierung (ca. 4 bis 5 Stunden) in 20 ml Eisessig erhitzt. Danach wird das gesamte Reaktionsgemisch im Vakuum eingeengt, in Essigsäureethylester aufgenommen, mit Wasser gewaschen und die organische Phase über Natriumsulfat getrocknet. Anschließend wird das Lösungsmittel im Vakuum abdestilliert und das zurückbleibende Rohprodukt über eine Kieselgelsäule (Kieselgel 60-Merck, Korngröße; 0,040 bis 0,063 mm) mit dem Fließmittel Methylenchlorid chromatographiert. Man erhält 1,3 g (31,5 % der Theorie) 3-(4-Chlor-phenoxymethyl)-5-phenyl-1,2,4-oxadiazol.
Fp.: 96 bis 102°C
¹H-NMR (CDCl₃),δ.ppm): 5,22 (s, 2H, -CH₂-O-); 6,99; 7,26 (2d, 4H, arom.-H; J = 9,0 Hz); 7,50 bis 8,17 (3m, 5H, arom.-H)

### Beispiel 7

### 3-(2,3-Dichlor-phenylthiomethyl)-5-methyl-1,2,4-oxadiazol

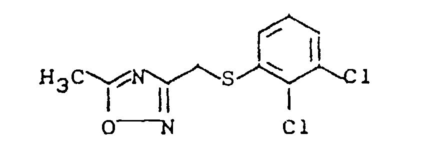

7,0 g (39,1 mMol) 2,3-Dichlor-thiophenol werden in 50 ml absolutem Tetrahydrofuran vorgelegt, portionsweise mit 1,2 g (0,039 mMol) 80%iger Natriumhydrid-Dispersion versetzt und 2 Stunden bei Rückflußtemperatur gerührt. Anschließend versetzt man bei Raumtemperatur mit 4,5 g (33,9 mMol) 3-Chlormethyl-5-methyl-1,2,4-oxadiazol und rührt die Mischung bis zum vollständigen Umsatz (ca. 10 bis 15 Stunden). Danach wird das ausgefallene Natriumchlorid abgetrennt und der gesamte Reaktionsansatz im Vakuum eingeengt. Das zurückbleibende Rohprodukt kann über eine Kieselgelsäule (Kieselgel 60-Merck, Korngröße: 0,040 bis 0,063 mm) mit dem Fließmittel Toluol chromatographiert werden. Man erhalt 3,8 g (36,5 % der Theorie) 3-(2,3-Dichlor-phenylthiomethyl)-5-methyl-1,2,4-oxadiazol.
Fp.: 66 bis 68°C
¹H-NMR (CDCl₃),δ.ppm): 2,57 (s, 3H, hetarom.-H); 4,17 (s, 2H, -CH₂-S-); 7,13-7,36 (m, 3H, arom.-H)

### Beispiel 8

### 3-(2,3-Dichlor-phenylsulfonylmethyl)-5-methyl-1,2,4-oxadiazol

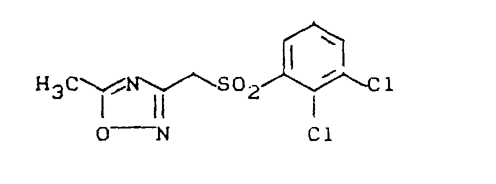

3,9 g (14,2 mMol) 3-(2,3-Dichlor-phenylthiomethyl)-5-methyl-1,2,4-oxadiazol werden in 50 ml Eisessig vorgelegt und mit 9,8 ml 30%igem Wasserstoffperoxid versetzt. Die Mischung wird bis zum vollständigen Umsatz (ca. 2 Stunden) bei Rückflußtemperatur gerührt. Danach wird der gesamte Reaktionsansatz in 200 ml Wasser eingetragen, das ausgefallene Rohprodukt abgetrennt und aus Isopropanol umkristallisiert. Man erhält 1,7 g (38,9 % der Theorie) 3-(2,3-Dichlor-phenylsulfonylmethyl)-5-methyl-1,2,4-oxadiazol.
Fp.: 105 bis 107°C
¹H-NMR (CDCl₃),δ.ppm); 2,55 (s, 3H, -CH₃); 4,86(s, 2H, -CH₂-SO₂-); 7,35 bis 7,95 (m, 3H, arom.-H)

Analog können die in der nachstehenden Tabelle 1 aufgeführten Verbindungen der Formel (I) hergestellt werden.

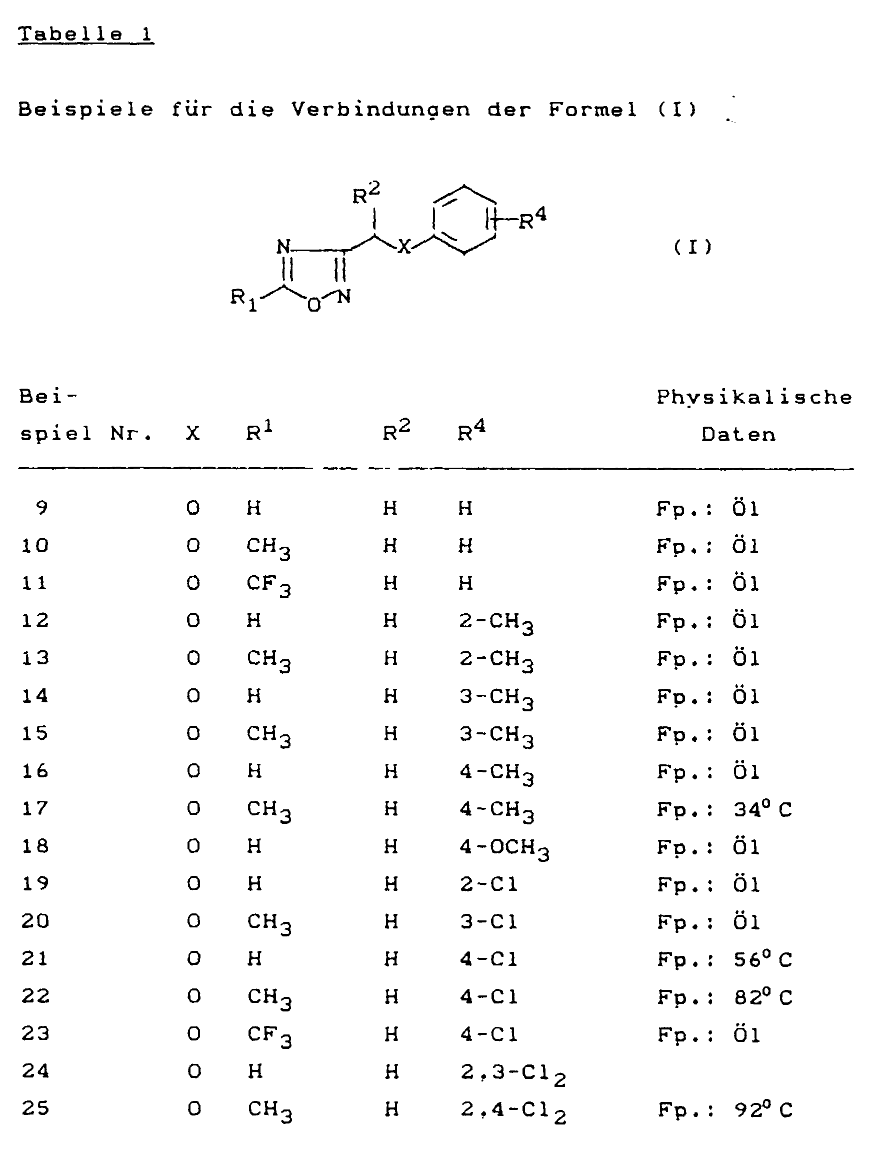

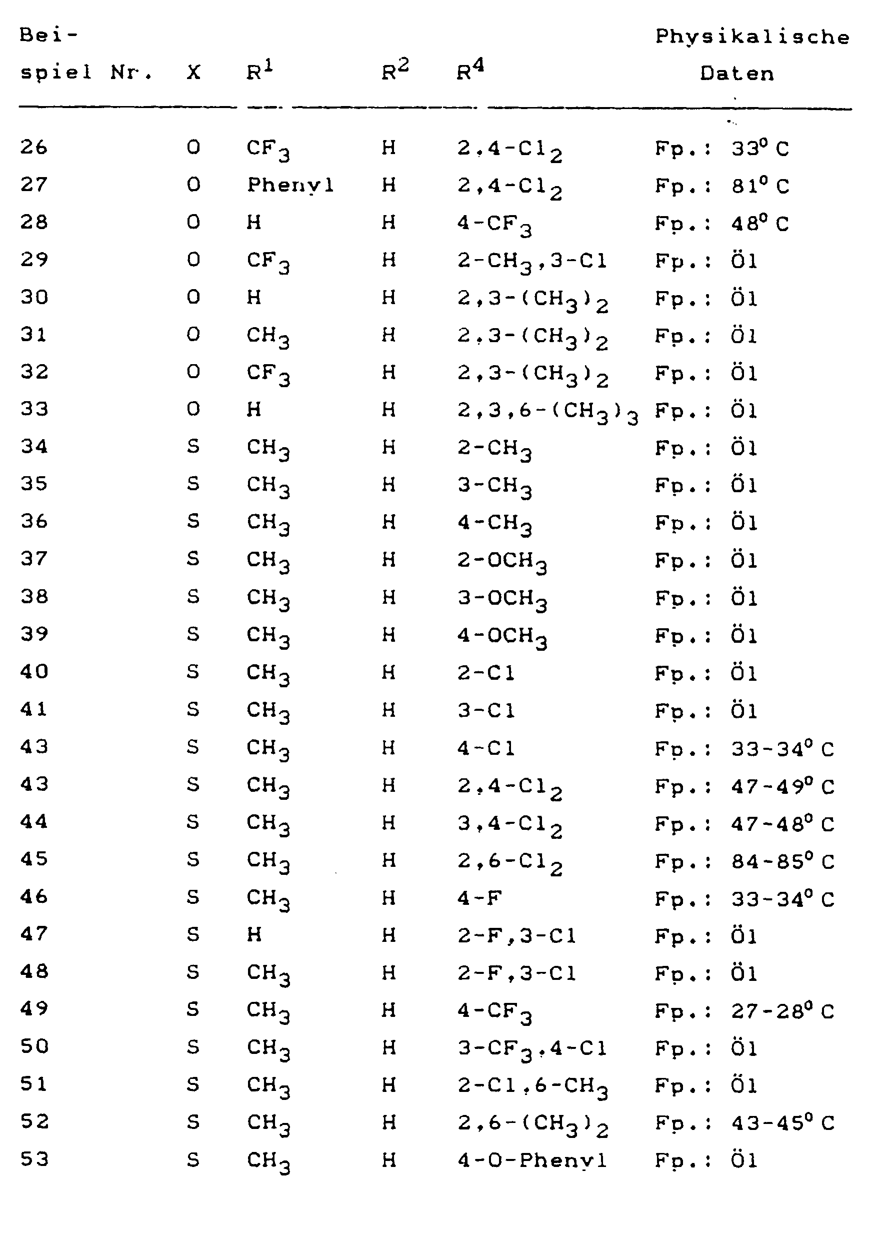

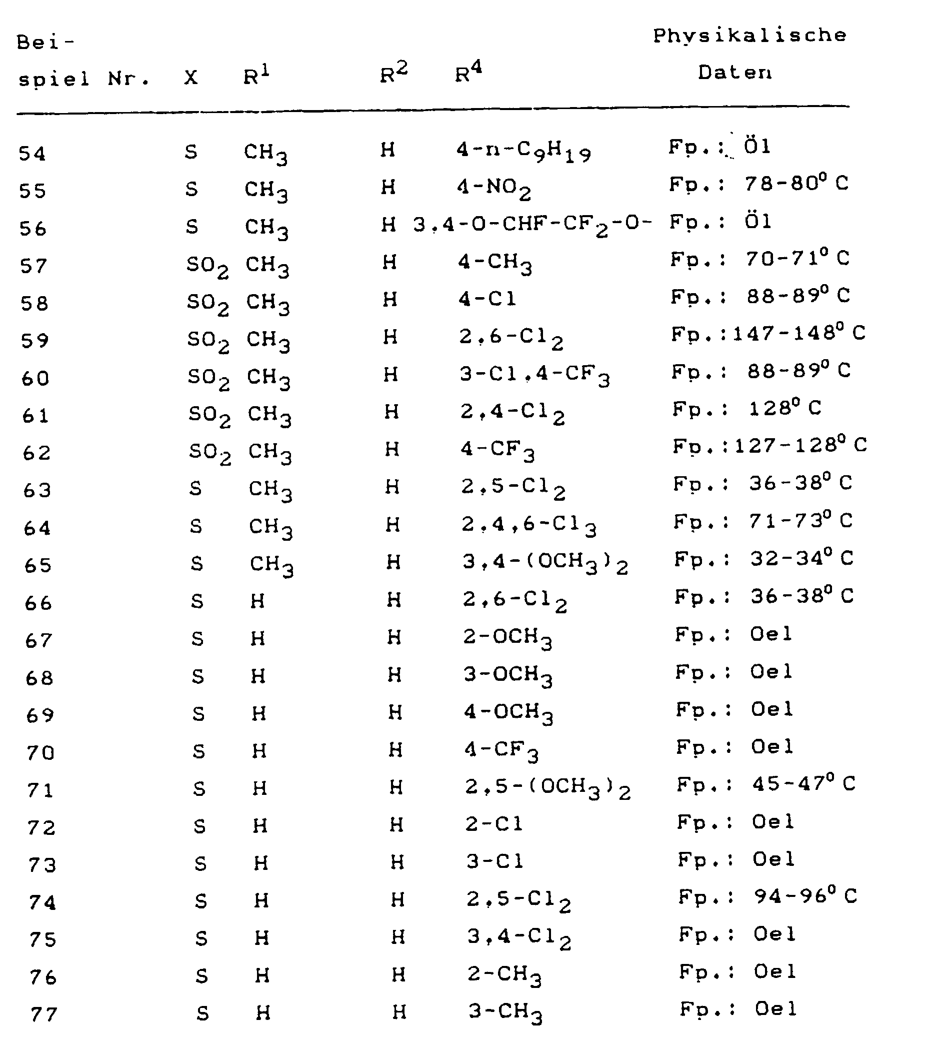

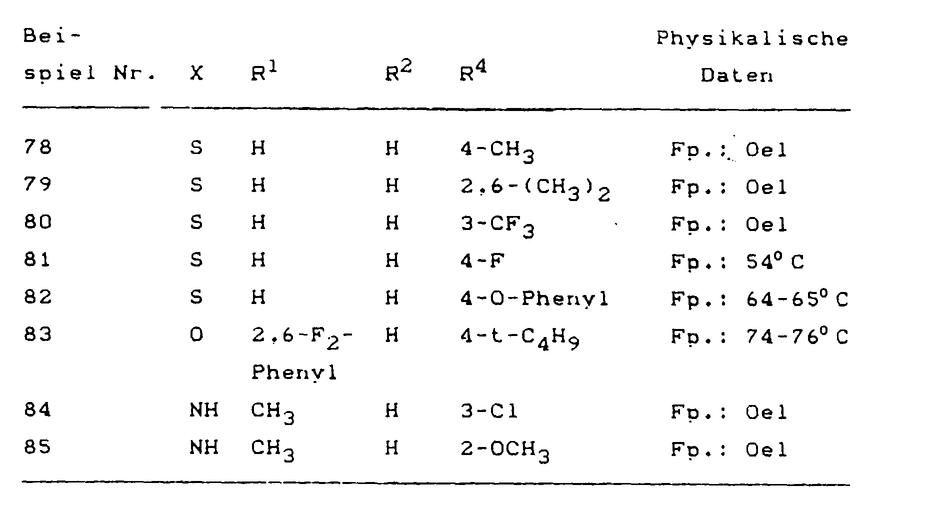

Ausgangsstoffe der Formel (II)

### Beispiel (II-1)

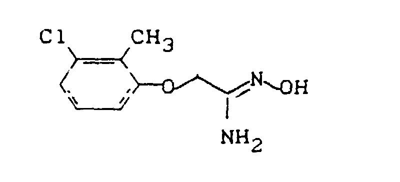

Zu einer Lösung aus 13,5 g (194,2 mMol) Hydroxylaminhydrochlorid, 7,7 g (194,2 mMol) Natriumhydroxid in 265 ml Ethanol, 68 ml Wasser werden 29,2 g (160,7 mMol) 3-Chlor-2-methyl-phenoxyacetonitril gegeben. Die Mischung wird bis zum vollständigen Umsatz (ca. 48 bis 60 Stunden) auf Rückflußtemperatur erhitzt und anschließend wird der gesamte Reaktionsansatz in 250 ml Wasser eingerührt. Der dabei abscheidende Feststoff wird abgetrennt, mit wenig Wasser gewaschen und getrocknet. Man erhält 31,1 g (90,2 % der Theorie) 3-Chlor-2-methylphenoxyacetamidoxim.

¹H-NMR (DMSO-d₆,δ,ppm): 2,23 (s, 3H, arom.-CH₃); 4,42 (s, 2H, -CH₂-O); 5,63 (s, 2H, -NH₂); 7,00-7,17 (m, 3H, arom.-H); 9,31 (s, 1H, =N-OH);
¹³C-NMR (DMSO-d₆,δ,ppm): 12,8 (-CH₃); 67,1 (-CH₂-O-); 110,9; 121,7 (arom.-C); 124,6 (arom.-CH₃); 133,9 (arom.-C=N-); 157,3 (arom.-C-O-)

### Beispiel (II-2)

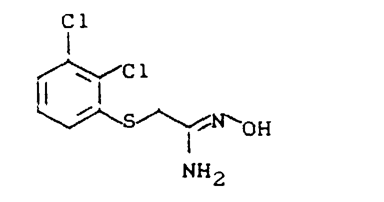

10,8 g (49,5 mMol) 2,3-Dichlor-phenylthioacetonitril, 7,3 g (105,0 mMol) Hydroxylamin-hydrochlorid und 11,1 g (105,0 mMol) Natriumcarbonat werden in 25 ml Ethanol, 50 ml Wasser bis zum vollständigen Umsatz (ca. 24 Stunden) bei Rückflußtemperatur gerührt. Anschließend wird der gesamte Reaktionsansatz in 200 ml Wasser eingetragen, der dabei abscheidende Feststoff abgesaugt, mit wenig Wasser gewaschen und getrocknet. Man erhält 12,2 g (98,1 % der Theorie) 2,3-Dichlor-phenylthioacetamidoxim.
¹H-NMR (DMSO-d₆,δ,ppm): 5,62 (s, 2H, -NH₂); 7,30-7,56 (m, 3H, arom.-H); 9,29 (br., 1H, =N-OH)

Analog können die in der nachstehenden Tabelle 2 aufgeführten Verbindungen der Formel (II) hergestellt werden.

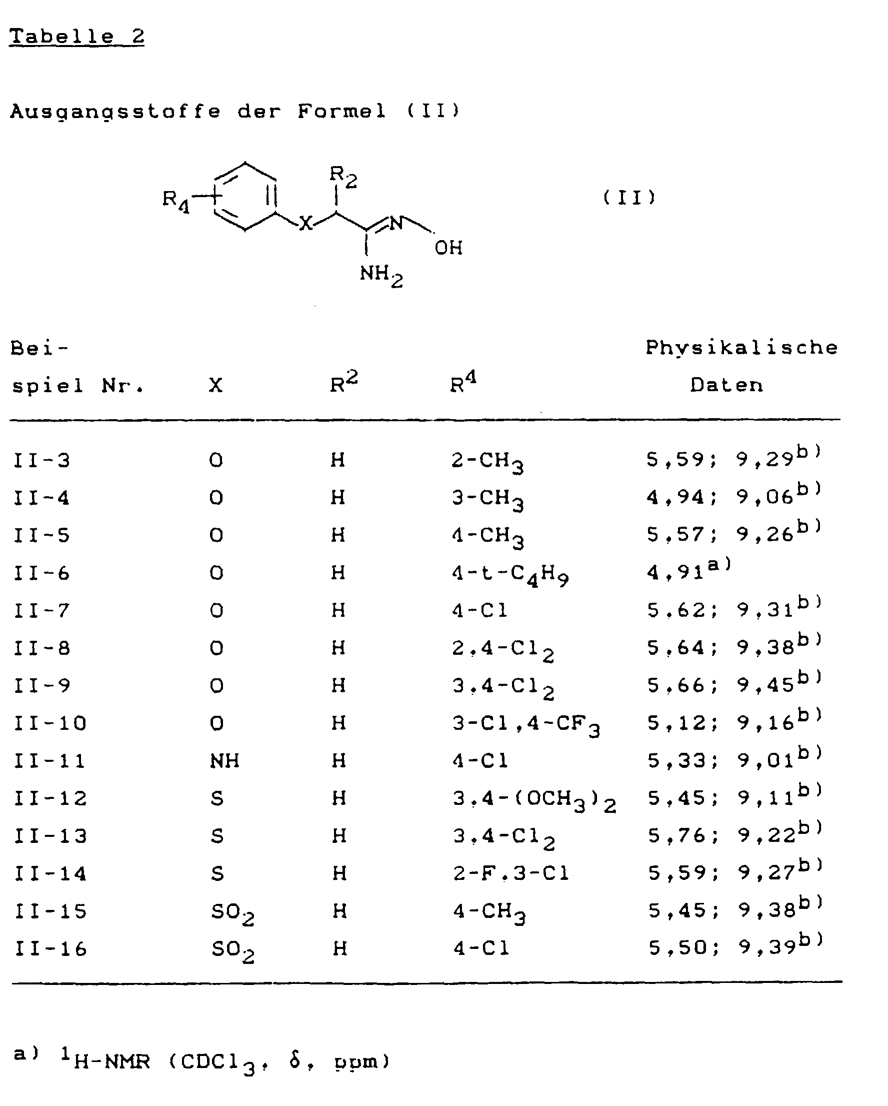

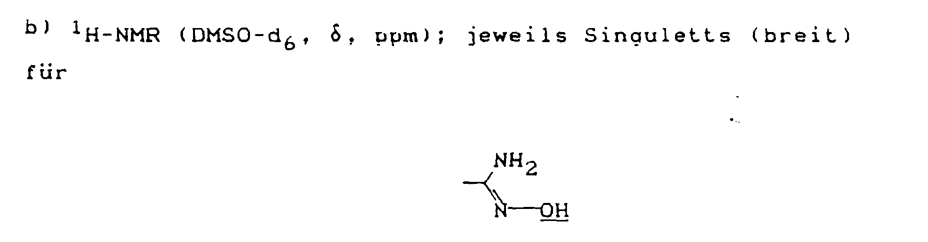

### Beispiel (VI-1)

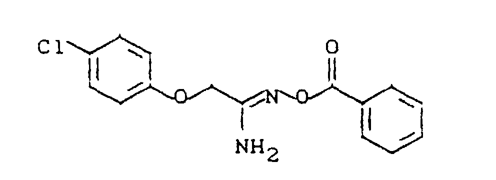

5,0 g (24,9 mMol) 4-Chlor-phenoxyacetamidoxim, 4,35 ml (27,7 mMol) N,N-Diisopropylethylamin ("Hünig's Base") werden in 50 ml Chloroform vorgelegt und bei Raumtemperatur tropfenweise mit 3,9 g (27,7 mMol) Benzoylchlorid versetzt. Nach zweistündigem Rühren wird der gesamte Reaktionsansatz mit Wasser gewaschen, über Natriumsulfat getrocknet und im Vakuum eingeengt. Anschließend wird das zurückbleibende Rohprodukt über eine Kieselgelsäule (Kieselgel 60-Merck, Korngröße: 0,040 bis 0,063 mm) mit dem Fließmittel Methylenchlorid: Methanol (100:1) chromatographiert. Man erhält 3,9 g (51,4 % der Theorie) O-Benzoyl-(4-chlor-phenoxy)-acetamidoxim.
Fp.: 149°C
¹H-NMR (CDCl₃,δ,ppm): 4,75 (s, 2H, -CH₂-O-); 5,25 (br. s, 2H, -NH₂); 6,93; 7,26 (2d, 4H, arom.-H; J = 9,1 Hz); 7,43-8,07 (3m, 5H, arom.-H)

Analog können die in der nachfolgenden Tabelle 3 aufgeführten Verbindungen der Formel (VI) hergestellt werden.

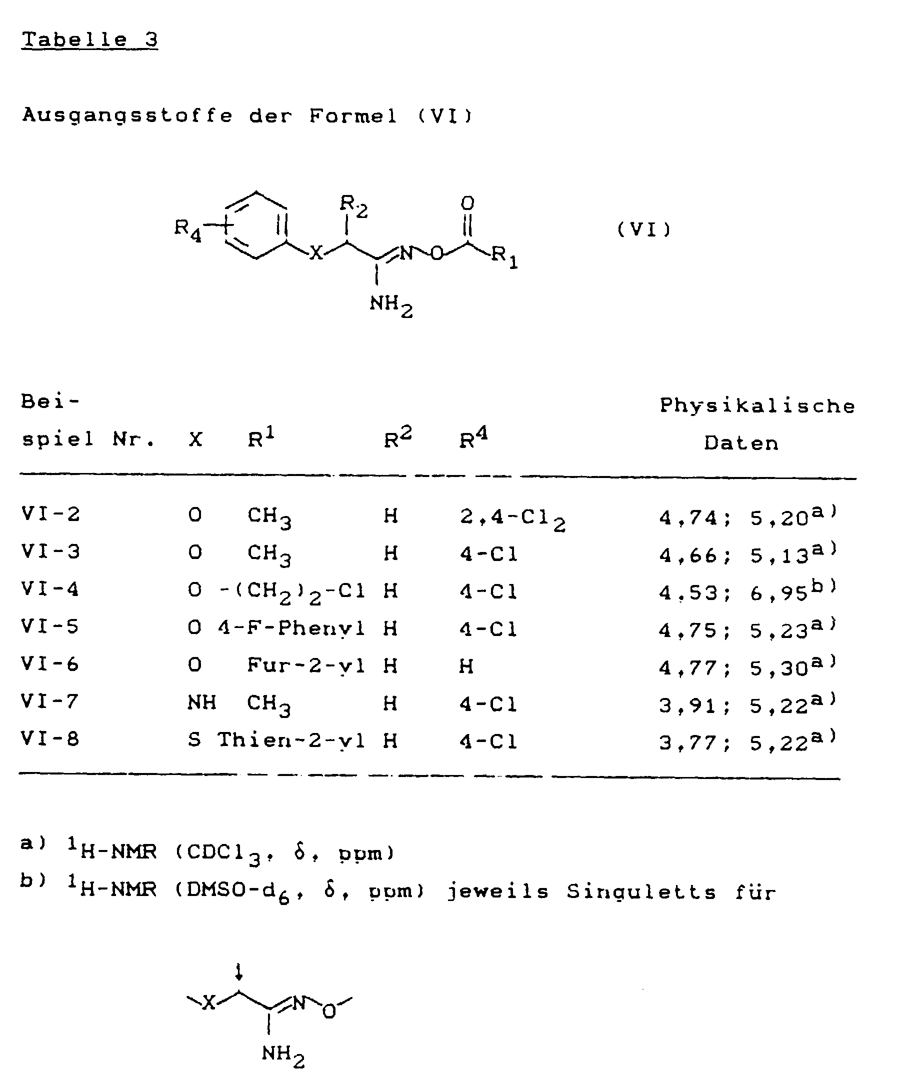

Beispiel zur Herstellung der als Vorstufe eingesetzten 5-substituierten 3-Halogenalkyl-1,2,4-Oxadiazole (vgl. DE-OS 2 406 786).

### Beispiel (VII-1)

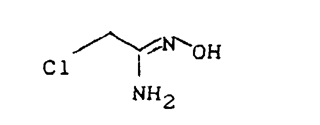

Zu einer Lösung aus 69,5 g (1,0 Mol) Hydroxylamin-hydrochlorid in 250 ml Wasser und 75,5 g (1,0 Mol) Chloracetonitril werden portionsweise 106,0 g (1,0 Mol) Natriumcarbonat gegeben; dabei sollte die Reaktionstemperatur nicht über 30°C ansteigen. Das Reaktionsgemisch wird noch 15 Minuten bei 30°C gerührt und anschließend mehrmals mit Ether extrahiert. Die organischen Phasen werden über Magnesiumsulfat getrocknet, im Vakuum eingeengt und mit Diisopropylether verrührt. Man erhält 54,6 g (50,3 % der Theorie) Chloressigsäureamidoxim.

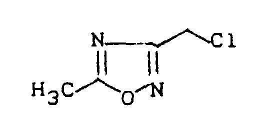

54,6 g (0,5 Mol) Chloressigsäureamidoxim werden in 96 ml Essigsäureanhydrid vorgelegt und 2 Stunden bei 120 bis 130°C gerührt. Anschließend wird der gesamte Reaktionsansatz im Vakuum eingeengt, mit Natriumhydrogencarbonat neutralisiert und mit Essigsäureethylester extrahiert.

Die organische Phase wird über Magnesiumsulfat getrocknet, das Lösungsmittel abdestilliert und der ölige Rückstand im Vakuum destilliert. Man erhält 27,3 g (41,2 % der Theorie) 3-Chlormethyl-5-methyl-1,2,4-oxadiazol.
Kp_{12mbar}: 60 bis 75°C
¹H-NMR(CDCl₃,δ,ppm); 2,63 (s, 3H, -CH₃); 4,59 (s, 2H, -CH₂-Cl)

Beispiel zur Herstellung der als Vorstufe eingesetzten substituierten Acetonitrile (vgl. I. Cervena et al. Collect.Czech.Chem.Commun.46(1981) 5, S. 1188-1198)

### Beispiel (IX-1)

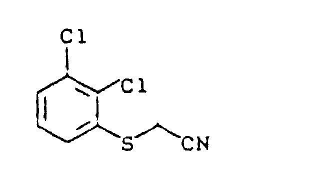

15,0 g (83,8 mMol) 2,3-Dichlor-thiophenol werden in 50 ml absolutem Methanol vorgelegt, unter Kühlung portionsweise mit 4,5 g (83,8 mMol) Natriummethanolat versetzt und 15 Minuten bei Raumtemperatur gerührt. Anschließend gibt man tropfenweise 6,3 g (83,8 mMol) Chloracetonitril hinzu, rührt 2 Stunden bei Raumtemperatur und weitere 5 Stunden bei Rückflußtemperatur. Danach wird der gesamte Reaktionsansatz heiß filtriert und im Vakuum eingeengt. Man erhält 10,8 g (59,1 % der Theorie) 2,3-Dichlor-phenylthioacetonitril.
¹H-NMR (DMSO-d₆,δ,ppm): 4,44 (s, 2H, -CH₂-S-); 6,87-7,59 (m, 3H, arom.-H)

Analog können die in der nachstehenden Tabelle 4 aufgeführten Verbindungen der Formel (IX) hergestellt werden.

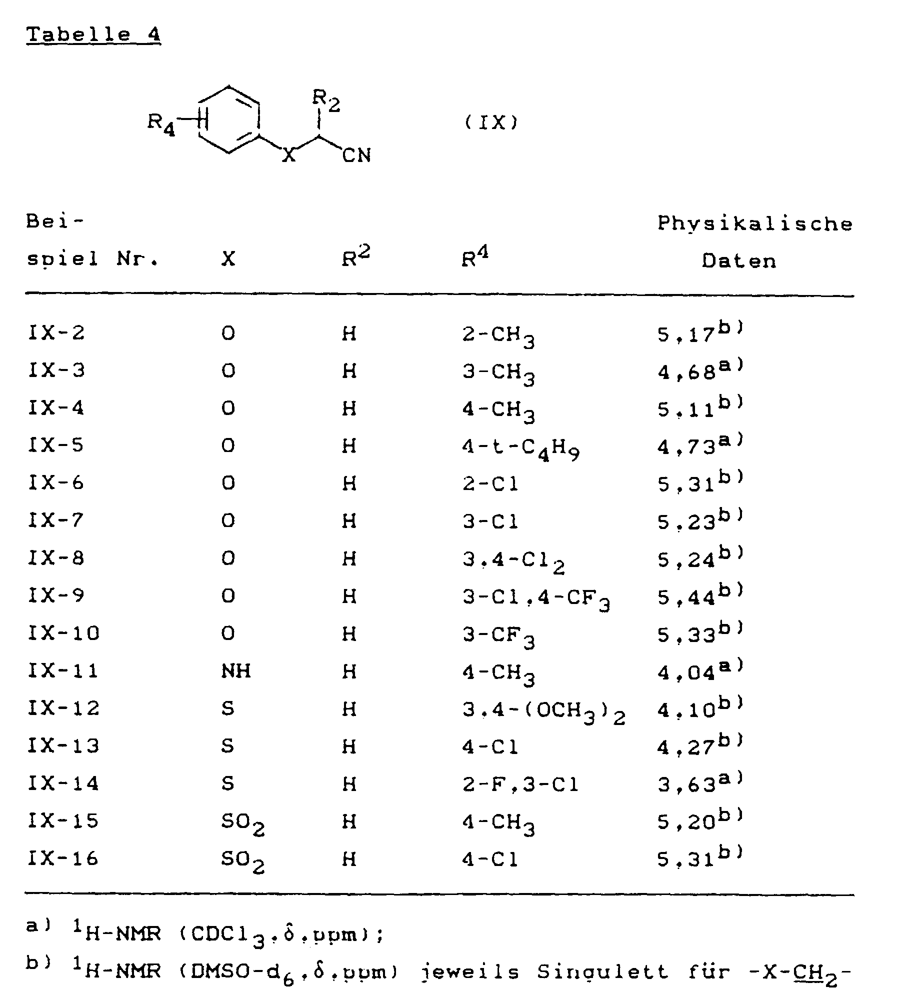

## Patentansprüche

1. Verwendung von substituierten 1,2,4-Oxadiazolderivaten der allgemeinen Formel (I) 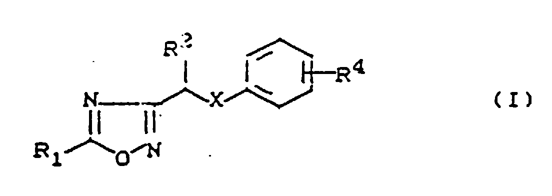 in welcher
R¹ für Wasserstoff, Alkyl das gegebenenfalls substituiert ist durch Halogen, Aikoxy, Hydroxy, Amino, Alkylamino, Dialkylamino, Cycloalkylamino sowie für Cycloalkyl, gegebenenfalls substituiertes Aryl oder Hetaryl steht,
R² mit Wasserstoff und Alkyl steht,
X für O, S, SO, SO₂ und N-R³ steht,
R³ für Wasserstoff, Alkyl, Alkylcarbonyl oder Halogenalkylcarbonyl steht,
R⁴ für gleiche oder verschiedene Reste aus der Reihe Wasserstoff, Halogen, Cyano, Nitro, Isothiocyanato, Amino, Aminoacyl, Alkylamino, Dialkylamino, Trialkylammonium-halogenid, Sulfonylamino, Hydroxy, Mercapto, Alkyl, Aralkyl, Halogenalkyl, Alkoxy, Aralkoxy, Halogenalkoxy, Aryl, Aryloxy, Arylthio, Arylsulfinyl, Arylsulfonyl, Alkylthio, Halogenalkylthio, Alkylsulfinyl, Halogenalkylsulfinyl, Alkylsulfonyl, Halogenalkylsulfonyl, Alkoxycarbonyl, Alkylcarbonyl, Alkylcarbonyloxy, Alkoxyalkyl, Alkoxyalkoxy, Hydroxyalkoxy, Hetarylmethylenoxy steht zur Herstellung von Mitteln gegen parasitäre Nematoden, Trematoden oder Cestoden.

2. Verwendung gemäß Anspruch 1 von substituierten 1,2,4-Oxadiazolderivaten der allgemeinen Formel (I) 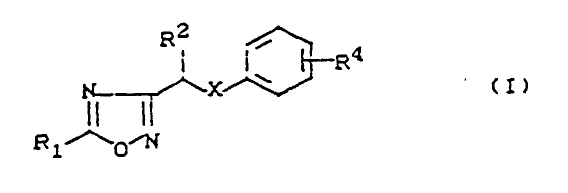 in welcher
R¹ für Wasserstoff, C₁-C₄-Alkyl das gegebenenfalls substituiert ist durch Halogen, für gegebenenfalls substituiertes Aryl oder Hetaryl, insbesondere Furyl, Thienyl sowie Pyridyl steht,
R² für Wasserstoff und C₁-C₄-Alkyl steht,
X für den Fall, daß R² für C₁₋₄-Alkyl steht für O, S, SO, SO₂ und N-R³ steht,
R³ und R⁴ die in Anspruch 1 angegebene Bedeutung besitzen,
zur Herstellung von Mitteln gegen Nematoden, Cestoden oder Trematoden.

3. Neue substituierte 1,2,4-Oxadiazolderivate der allgemeinen Formel (I) 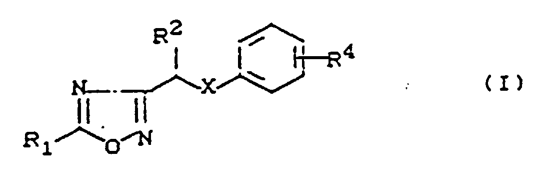 in welcher
R¹ für Wasserstoff, C₁-C₄-Alkyl das gegebenenfalls substituiert ist durch Halogen, gegebenenfalls substituiertes Aryl oder Hetaryl, insbesondere Furyl, Thienyl sowie Pyridyl steht,
R² für Wasserstoff steht,
X für S steht,
R⁴ für 2-Cl; 3-Cl; 2,3-Cl₂: 2,4-Cl₂; 2,5-Cl₂, 2,6-Cl₂, 2,4,6-Cl₃; 2-F, 3-Cl; 2,3-F₂; 3-Br; 3,4-OCHF-CF₂-O; 2-OCF₃; 4-OCF₃; 4-CF₃, 3,5-(CF₃)₂; 3,5-(CH₃)₂; 3,4-(OCH₃)₂; 2,5-(OCH₃)₂; 4-nC₉H₁₉; 4-N(C₂H₅)₂; 4-NH-CH₃; 4-NH-C₂H₅; 4-NH-CO-CH₃; 4-N⁺(CH₃)₃Cl⁻; 4-N⁺(CH₃)₃I⁻ steht,
wobei R⁴ für den Fall, daß R¹ eine andere Bedeutung als Wasserstoff hat, zusätzlich für 3,4-Cl₂; 2,4,5-Cl₃; 4-F; 2-Br; 3-CF₃; 2-CH₃; 3-CH₃; 2,4-(CH₃)₂; 2,6(CH₃)₂; 4-OH; 4-O-OC-CH₃; 2-OCH₃; 3-OCH₃; 4-OCH₃; 4-tC₄H₉; 4-NCS; 4-NH₂; 4-NH-CH₃; 4-N(CH₃)₂; 4-N(C₂H₅)₂ steht,
sowie die Verbindungen der Formel (IA)

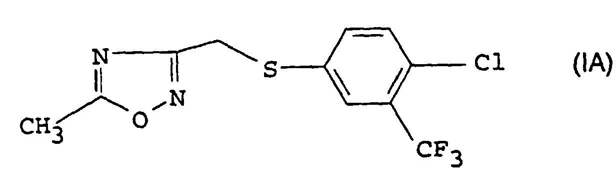

4. Verfahren zur Herstellung der neuen substituierten 1,2,4-Oxadiazolderivate der Formel (I) und (IA) gemäß Anspruch 3 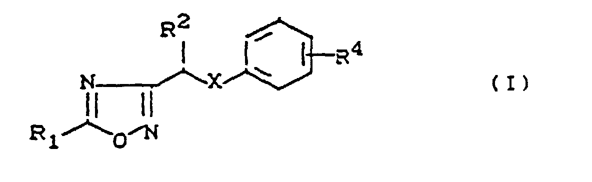 in welcher
X, R¹, R², R³ und R⁴ die in Anspruch 3 angegebene Bedeutung besitzen,
dadurch gekennzeichnet, daß man
a) Verbindungen der Formel (II) 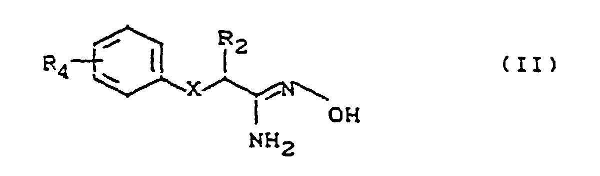 in welcher
X, R², R³ und R⁴ die oben angegebene Bedeutung besitzen,
mit Carbonsäurederivaten der Formeln (III), (IV) oder (V)
R¹-C(O-R)₃ (III)
(R¹-CO)₂O (IV)
R¹-CO-Y (V)
worin
R¹ die oben angegebene Bedeutung hat und R für Methyl oder Ethyl steht, Y für eine geeignete Abgangsgruppe steht, umsetzt oder
b ) Verbindungen der Formel (VI) 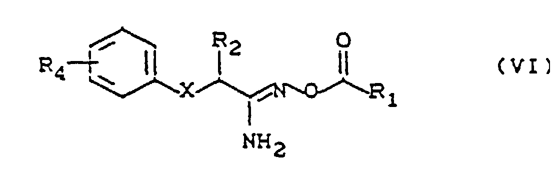 in welcher
X, R¹, R², R³ und R⁴ die oben angegebene Bedeutung besitzen,
gegebenenfalls in Gegenwart eines Verdünnungsmittels und gegebenenfalls in Gegenwart eines Reaktionshilfsmittels cyclisiert, oder
c) Verbindungen der Formel (VII) 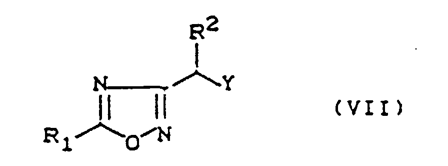 in welcher
R¹ und R² die oben angegebene Bedeutung haben,
Y für eine geeignete Abgangsgruppe steht, mit Verbindungen der Formel (VIII) 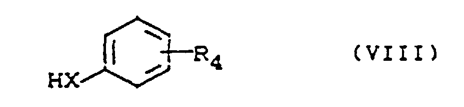
in welcher
X und R⁴ die oben angegebene Bedeutung besitzen,
in Gegenwart eines Verdünnungsmittels und gegebenenfalls in Gegenwart eines Reaktionshilfsmittels umsetzt.

5. Verwendung gemäß Anspruch 1 von Verbindungen der Formel (I) in welcher
R¹ für Wasserstoff, C₁-C₄-Alkyl das gegebenenfalls substituiert ist durch Fluor, Chlor oder Brom, C₁-C₄-Alkoxy, Hydroxy, Amino, C₁-C₄-Alkylamino, C₁-C₄-Dialkylamino, sowie Cyclo-C₃-C₇-alkylamino steht, R¹ steht ferner für Cyclo-C₃-C₇-alkyl, gegebenenfalls substituiertes Phenyl, Furyl, Thienyl der Pyridyl;
R² für Wasserstoff und Methyl steht,
X für O, S, SO, SO₂ und -N-R³ steht,
R³ für Wasserstoff, C₁-C₄-Alkyl, C₁-C₄-Alkylcarbonyl, Halogen-C₁-C₄-alkyl, steht,
R⁴ für gleiche oder verschiedene Reste aus der Reihe Wasserstoff, Fluor, Chlor oder Brom, Cyano, Nitro, Isothiocyanato, Amino, C₁-C₄-Alkylamino, C₁-C₄-Dialkylamino, C₁-C₄-Trialkylammonium-halogenid, Sulfonylamino, Hydroxy, Mercapto, C₁-C₉-Alkyl, Aralkyl, Halogen-C₁-C₄-Alkyl, C₁-C₄-Alkoxy, Methylendioxy oder Ethylendioxy, die gegebenenfalls durch Fluor oder Chlor substituiert sind, Halogen-C₁-C₄-alkoxy, Aralkoxy, Aryl, Aryloxy, Arylthio, Arylsulfinyl, Arylsulfonyl, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Alkoxycarbonyl, Alkylcarbonyl, sowie Oxadiazolylmethylenoxy das durch einen der bei R¹ angegebenen Reste substituiert ist, steht, zur Herstellung von Mitteln gegen Nematoden, Trematoden oder Cestoden.

6. Verwendung gemäß Anspruch 1 von Verbindungen der Formel (1), in welcher
R¹ für Wasserstoff, Methyl, ethyl, Isopropyl oder sec.Butyl, Trichlormethyl oder Trifluormethyl steht,
R² für Wasserstoff oder Methyl steht,
X für O, S, SO, SO₂ und -N-R³ steht,
R³ für Wasserstoff, Acetyl, Propionyl oder Trifluoracetyl steht,
R⁴ für gleiche oder verschiedene Reste aus der Reihe für Wasserstoff, Fluor, Chlor, Brom, Nitro, Isothiocyanato, Amino, Methylamino, Ethylamino, Dimethylamino, Trimethyl-ammonium-bromid, -chlorid oder-iodid, Hydroxy, Mercapto, Methyl, Ethyl, Isopropyl, Butyl, Hexyl, Oktyl oder Nonyl, Trifluormethyl, Methoxy, Ethoxy, Methylendioxy oder Ethylendioxy, die gegebenenfalls durch Fluor oder Chlor substituiert sind, Trifluormethoxy, Flurochlorethoxy, Methylthio, Trifluormethylthio, Fluorchlormethylthio, Methylsulfinyl, Trifluormethylsulfinyl, Methylsulfonyl, Trifluormethylsulfonyl sowie für 3-(1 ,2,4-Oxadiazolyl)-methylendioxy, das durch einen der bei R¹ angegebenen Reste in 5-Stellung substituiert ist, steht,
zur Herstellung von Mitteln gegen Nematoden, Trematoden oder Cestoden.

7. Mittel gegen Nematoden, Cestoden oder Trematoden gekennzeichnet durch einen Gehalt an mindestens einem substituierten 1,2,4-Oxadiazolderivat der Formel (I) gemäß Anspruch 3.

8. Verwendung von substituierten 1,2,4-Oxadiazolderivaten der Formel (I) gemäß Anspruch 3 zur Herstellung von Mitteln gegen Nematoden, Cestoden oder Trematoden.

## Claims

1. Use of substituted 1,2,4-oxadiazole derivatives of the general formula I 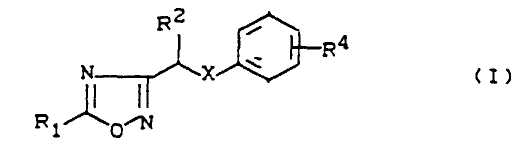 in which
R¹ represents hydrogen, alkyl which is optionally substituted by halogen, alkoxy, hydroxyl, amino, alkylamino, dialkylamino, cycloalkylamino as well as represents cycloalkyl, optionally substituted aryl or hetaryl,
R² represents hydrogen and alkyl,
X represents O, S, SO, SO₂ and N-R³,
R³ represents hydrogen, alkyl, alkylcarbonyl or halogenoalkylcarbonyl,
R⁴ represents identical or different radicals from the series comprising hydrogen, halogen, cyano, nitro, isothiocyanato, amino, aminoacyl, alkylamino, dialkylamino, trialkylammonium halide, sulphonylamino, hydroxyl, mercapto, alkyl, aralkyl, halogenoalkyl, alkoxy, aralkoxy, halogenoalkoxy, aryl, aryloxy, arylthio, arylsulphinyl, arylsulphonyl, alkylthio, halogenoalkylthio, alkylsulphinyl, halogenoalkylsulphinyl, alkylsulphonyl, halogenoalkylsulphonyl, alkoxycarbonyl, alkylcarbonyl, alkylcarbonyloxy, alkoxyalkyl, alkoxyalkoxy, hydroxyalkoxy, hetarylmethyleneoxy,
for the preparation of compositions against parasitical nematodes, trematodes or cestodes.

2. Use in accordance with Claim 1 of substituted 1,2,4-oxadiazole derivatives of the general formula (I) 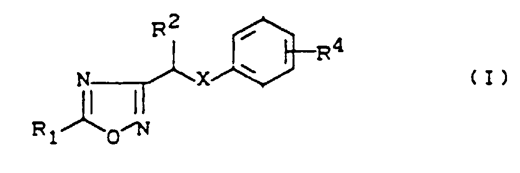 in which
R¹ represents hydrogen, C₁-C₄-alkyl which is optionally substituted by halogen, or represents optionally substituted aryl or hetaryl, in particular furyl, thienyl as well as pyridyl,
R² represents hydrogen and C₁-C₄-alkyl,
X, in the event that R² represents C₁₋₄-alkyl, represents O, S, SO, SO₂ and N-R³,
R³ and R⁴ have the meaning given in Claim 1,
for the preparation of compositions against nematodes, cestodes or trematodes.

3. New substituted 1,2,4-oxadiazole derivatives of the general formula (I) 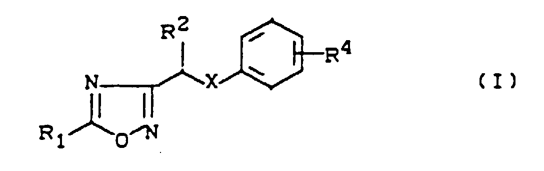 in which
R¹ represents hydrogen, C₁-C₄-alkyl which is optionally substituted by halogen, optionally substituted aryl or hetaryl, in particular furyl, thienyl as well as pyridyl,
R² represents hydrogen,
X represents S,
R⁴ represents 2-Cl; 3-Cl; 2,3-Cl₂; 2,4-Cl₂; 2,5-Cl₂, 2,6-Cl₂, 2,4,6-Cl₃; 2-F, 3-Cl; 2,3-F₂; 3-Br; 3,4-OCHF-CF₂-O; 2-OCF₃; 4-OCF₃; 4-CF₃, 3,5-(CF₃)₂; 3,5-(CH₃)₂; 3,4-(OCH₃)₂; 2,5-(OCH₃)₂; 4-nC₉H₁₉; 4-N(C₂H₅)₂; 4-NH-CH₃; 4-NH-C₂H₅; 4-NH-CO-CH₃; 4-N⁺(CH₃)₃Cl⁻; 4-N⁺(CH₃)₃I⁻,
where R⁴ in the event that R¹ has a meaning other than hydrogen, additionally represents 3,4-Cl₂; 2,4,5-Cl₃; 4-F; 2-Br; 3-CF₃; 2-CH₃; 3-CH₃; 2,4-(CH₃)₂; 2,6(CH₃)₂; 4-OH; 4-O-OC-CH₃; 2-OCH₃; 3-OCH₃; 4-OCH₃; 4-tC₄H₉; 4-NCS; 4-NH₂; 4-NH-CH₃; 4-N(CH₃)₂; 4-N(C₂H₅)₂,
as well as the compounds of the formula (IA)

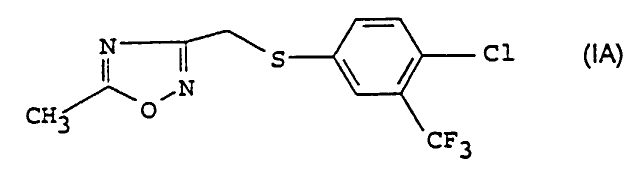

4. Processes for the preparation of the new substituted 1,2,4-oxadiazole derivatives of the formula (I) and (IA) according to Claim 3 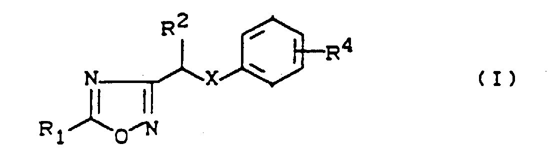 in which
X, R¹, R², R³ and R⁴ have the meaning given in Claim 3,
characterised in that
a) compounds of the formula (II) 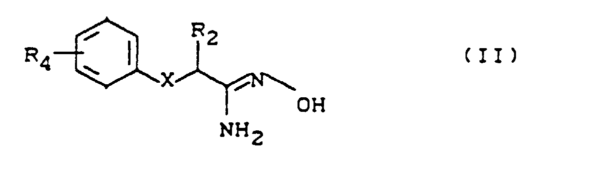 in which
X, R², R³ and R⁴ have the abovementioned meaning,
are reacted with carboxylic acid derivatives of the formulae (III), (IV) or (V)
R¹-C(O-R)₃ (III)
(R¹-CO)₂O (IV)
R¹-CO-Y (V)
in which
R¹ has the abovementioned meaning and R represents methyl or ethyl, and Y represents a suitable leaving group, or
b) compounds of the formula (VI) 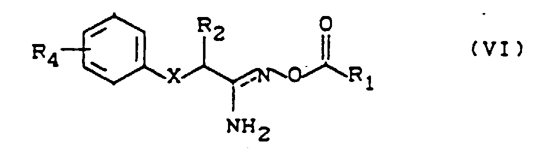 in which
X, R¹, R², R³ and R⁴ have the abovementioned meaning,
are cyclised, if appropriate in the presence of a diluent and if appropriate in the presence of a reaction auxiliary, or
c) compounds of the formula (VII) 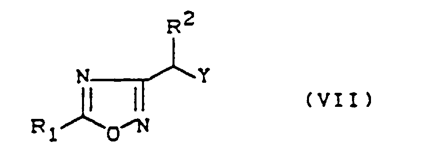 in which
R¹ and R² have the abovementioned meaning, and
Y represents a suitable leaving group are reacted with compounds of the formula (VIII) 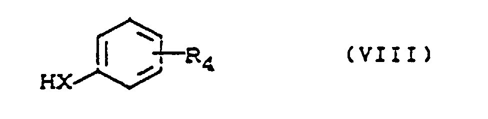
in which
X and R⁴ have the abovementioned meaning, in the presence of a diluent and if appropriate in the presence of a reaction auxiliary.

5. Use according to Claim 1 of compounds of the formula (I) in which
R¹ represents hydrogen, C₁-C₄-alkyl which is optionally substituted by fluorine, chlorine or bromine, C₁-C₄-alkoxy, hydroxyl, amino, C₁-C₄-alkylamino, C₁-C₄-dialkylamino as well as cyclo-C₃-C₇-alkylamino, R¹ furthermore represents cyclo-C₃-C₇-alkyl, optionally substituted phenyl, furyl, thienyl or pyridyl,
R² represents hydrogen and methyl,
X represents O, S, SO, SO₂ and -N-R³,
R³ represents hydrogen, C₁-C₄-alkyl, C₁-C₄-alkylcarbonyl, halogeno-C₁-C₄-alkyl,
R⁴ represents identical or different radicals from the series comprising hydrogen, fluorine, chlorine or bromine, cyano, nitro, isothiocyanato, amino, C₁-C₄-alkylamino, C₁-C₄-dialkylamino, C₁-C₄-trialkylammonium halide, sulphonylamino, hydroxyl, mercapto, C₁-C₉-alkyl, aralkyl, halogeno-C₁-C₄-alkyl, C₁-C₄-alkoxy, methylenedioxy or ethylenedioxy, each of which is optionally substituted by fluorine or chlorine, halogeno-C₁-C₄-alkoxy, aralkoxy, aryl, aryloxy, arylthio, arylsulphinyl, arylsulphonyl, C₁-C₄-alkylthio, C₁-C₄-halogenoalkylthio, C₁**-**C₄-alkylsulphinyl, C₁-C₄-halogenoalkylsulphinyl, C₁-C₄-alkylsulphonyl, C₁-C₄-halogenoalkylsulphonyl, alkoxycarbonyl, alkylcarbonyl, as well as oxadiazolylmethyleneoxy which is substituted by one of the radicals indicated for R¹, for the preparation of compositions against nematodes, trematodes or cestodes.

6. Use according to Claim 1 of compounds of the formula (I) in which
R¹ represents hydrogen, methyl, ethyl, isopropyl or sec. butyl, trichloromethyl or trifluoromethyl,
R² represents hydrogen or methyl,
X represents O, S, SO, SO₂ and -N-R³,
R³ represents hydrogen, acetyl, propionyl or trifluoroacetyl,
R⁴ represents identical or different radicals from the series comprising hydrogen, fluorine, chlorine, bromine, nitro, isothiocyanato, amino, methylamino, ethylamino, dimethylamino, trimethylammonium bromide, trimethylammonium chloride or trimethylammonium iodide, hydroxyl, mercapto, methyl, ethyl, isopropyl, butyl, hexyl, octyl or nonyl, trifluoromethyl, methoxy, ethoxy, methylenedioxy or ethylenedioxy, each of which is optionally substituted by fluorine or chlorine, trifluoromethoxy, fluorochloroethoxy, methylthio, trifluoromethylthio, fluorochloromethylthio, methylsulphinyl, trifluoromethylsulphinyl, methylsulphonyl, trifluoromethylsulphonyl, as well as 3-(1,2,4-oxadiazolyl)-methylenedioxy, which is substituted in the 5-position by one of the radicals given under R¹,
for the preparation of compositions against nematodes, trematodes or cestodes.

7. Compositions against nematodes, cestodes or trematodes, characterised by a content of at least one substituted 1,2,4-oxadiazole derivative of the formula (I) according to Claim 3.

8. Use of substituted 1,2,4-oxadiazole derivatives of the formula (I) according to Claim 3 for the preparation of compositions against nematodes, cestodes or trematodes.

## Revendications

1. Utilisation de dérivés substitués de 1,2,4-oxadiazole de formule générale (I) 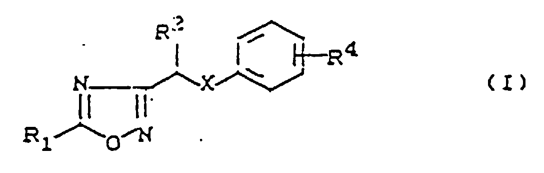 dans laquelle
R¹ représente de l'hydrogène, un groupe alkyle qui est substitué, le cas échéant par un halogène, un radical alkoxy, hydroxy, amino, alkylamino, dialkylamino, cycloalkylamino, ainsi qu'un groupe cycloalkyle éventuellement substitué par un radical aryle ou hétaryle,
R² représente de l'hydrogène ou un groupe alkyle,
X représente O, S, SO, SO₂ et N-R³,
R³ est de l'hydrogène, un groupe alkyle, alkylcarbonyle ou halogénalkylcarbonyle,
R⁴ représente des restes identiques ou différents de la série hydrogène, halogéno, cyano, nitro, isothiocyanato, amino, aminoacyle, alkylamino, dialkylamino, halogénure de trialkylammonium, sulfonylamino, hydroxy, mercapto, alkyle, aralkyle, halogénalkyle, alkoxy, aralkoxy, halogénalkoxy, aryle, aryloxy, arylthio, arylsulfinyle, arylsulfonyle, alkylthio, halogénalkylthio, alkylsulfinyle, halogénalkylsulfinyle, alkylsulfonyle, halogénalkylsulfonyle, alkoxycarbonyle, alkylcarbonyle, alkylcarbonyloxy, alkoxyalkyle, alkoxyalkoxy, hydroxyalkoxy, hétarylméthylèneoxy,
pour la préparation de compositions destinées à combattre des nématodes, des trématodes ou des cestodes parasites.

2. Utilisation suivant la revendication 1 de dérivés substitués de 1,2,4-oxadiazole de formule générale (I) 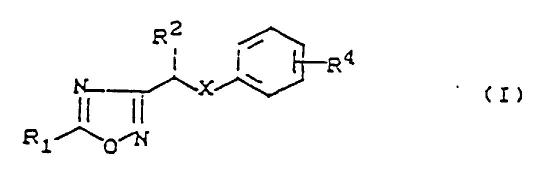 dans laquelle
R¹ est de l'hydrogène, un groupe alkyle en C₁ à C₄ qui est substitué, le cas échéant par un halogène, un groupe aryle ou hétaryle, notamment furyle, thiényle ainsi que pyridyle éventuellement substitué,
R² est de l'hydrogène et un groupe alkyle en C₁ à C₄,
X au cas où R² est un groupe alkyle en C₁ à C₄, représente O, S, SO, SO₂ et N-R³,
R³ et R⁴ ont la définition indiquée dans la revendication 1,
pour la préparation de compositions destinées à combattre des nématodes, des cestodes ou des trématodes.

3. Dérivés substitués nouveaux de 1,2,4-oxadiazole de formule générale (I) 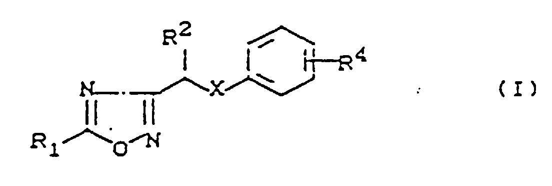 dans laquelle
R¹ représente de l'hydrogène, un groupe alkyle en C₁ à C₄ qui est substitué, le cas échéant par un halogène, un groupe aryle ou hétaryle, notamment furyle, thiényle ainsi que pyridyle, éventuellement substitué,
R² représente de l'hydrogène,
X représente S,
R⁴ représente 2-Cl; 3-Cl; 2,3-Cl₂; 2,4-Cl₂; 2,5-Cl₂, 2,6-Cl₂, 2,4,6-Cl₃; 2-F, 3-Cl; 2,3-F₂; 3-Br; 3,4-OCHF-CF₂-O; 2-OCF₃; 4-OCF₃; 4-CF₃, 3,5-(CF₃)₂; 3,5-(CH₃)₂; 3,4-(OCH₃)₂; 2,5-(OCH₃)₂; 4-nC₉H₁₉; 4-N(C₂H₅)₂; 4-NH-CH₃; 4-NH-C₂H₅; 4-NH-CO-CH₃; 4-N⁺(CH₃)₃Cl⁻; 4-N⁺(CH₃)₃I⁻,
où R⁴, au cas où R¹ a une définition autre que l'hydrogène, représente en outre 3,4-Cl₂; 2,4,5-Cl₃; 4-F; 2-Br; 3-CF₃; 2-CH₃; 3-CH₃; 2,4-(CH₃)₂; 2,6(CH₃)₂; 4-OH; 4-O-OC-CH₃; 2-OCH₃; 3-OCH₃; 4-OCH₃; 4-tC₄H₉; 4-NCS; 4-NH₂; 4-NH-CH₃; 4-N(CH₃)₂; 4-N(C₂H₅)_{2,}
ainsi que les composés de formule (IA)

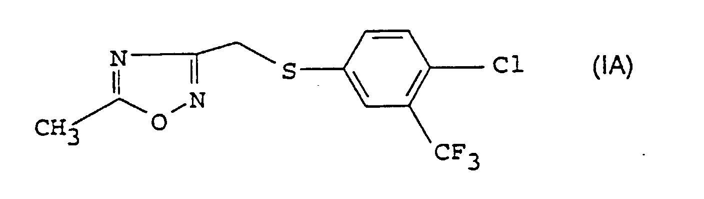

4. Procédé de production des dérivés substitués nouveaux de 1,2,4-oxadiazole de formules (I) et (IA) suivant la revendication 3 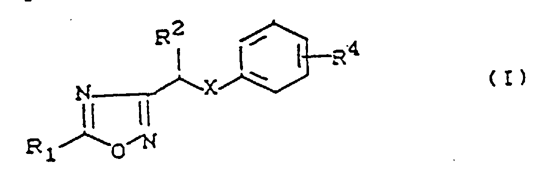 dans laquelle
X, R¹, R², R³ et R⁴ ont la définition donnée dans la revendication 3,
caractérisé en ce que
a) on fait réagir des composés de formule (II) 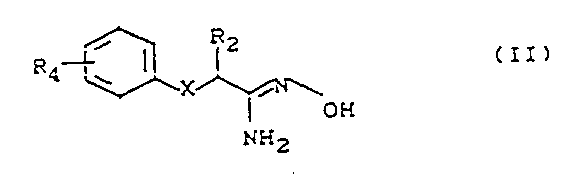 dans laquelle
X, R², R³ et R⁴ ont la définition indiquée ci-dessus,
avec des dérivés d'acides carboxyliques de formules (III), (IV) ou (V)
R¹-C(O-R)₃ (III)
(R¹-CO)₂O (IV)
R¹-CO-Y (V)
dans lesquelles
R¹ a la définition indiquée ci-dessus et R est un groupe méthyle ou éthyle, Y est un groupe partant approprié, ou bien
b) on cyclise des composés de formule (VI) 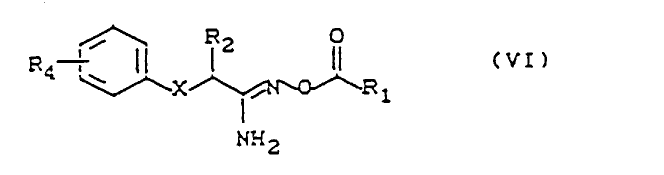 dans laquelle
X, R¹, R², R³ et R⁴ ont la définition indiquée ci-dessus, le cas échéant en présence d'un diluant et en la présence éventuelle d'une substance auxiliaire de réaction, ou bien
c) on fait réagir des composés de formule (VII) 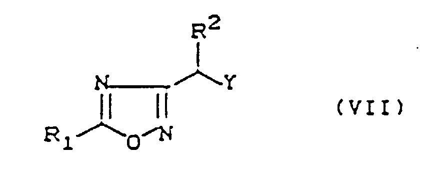 dans laquelle
R¹ et R² ont la définition indiquée ci-dessus,
Y est un groupe partant approprié,
avec des composés de formule (VIII) 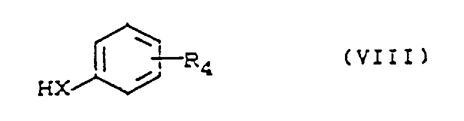 dans laquelle
X et R⁴ ont la définition indiquée ci-dessus,
en présence d'un diluant et en la présence éventuelle d'une substance auxiliaire de réaction.

5. Utilisation suivant la revendication 1, de composés de formule (I) dans laquelle
R¹ représente de l'hydrogène, un groupe alkyle en C₁ à C₄ qui est substitué, le cas échéant, par du fluor, du chlore ou du brome, un groupe alkoxy en C₁ à C₄, hydroxy, amino, alkylamino en C₁ à C₄, di-(alkyle en C₁ à C₄)amino ainsi que (cycloalkyle en C₃ à C₇)amino, R¹ représente en outre un groupe cycloalkyle en C₃ à C₇, phényle éventuellement substitué, furyle, thiényle ou pyridyle ;
R² représente de l'hydrogène et un groupe méthyle,
X représente O, S, SO, SO₂ et -N-R³,
R³ est de l'hydrogène, un groupe alkyle en C₁ à C₄, (alkyle en C₁ à C₄)-carbonyle, halogénalkyle en C₁ à C₄,
R⁴ représente des restes identiques ou différents de la série hydrogène, fluor, chlore ou brome, cyano, nitro, isothiocyanato, amino, alkylamino en C₁ à C₄, di-(alkyle en C₁ à C₄)amino, halogénure de tri-(alkyle en C₁ à C₄)ammonium sulfonylamino, hydroxy, mercapto, alkyle en C₁ à C₉, aralkyle, halogénalkyle en C₁ à C₄, alkoxy en C₁ à C₄, méthylènedioxy ou éthylènedioxy qui sont substitués, le cas échéant, par du fluor ou du chlore, halogénalkoxy en C₁ à C₄, aralkoxy, aryle, aryloxy, arylthio, arylsulfinyle, arylsulfonyle, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄, alkoxycarbonyle, alkylcarbonyle, ainsi qu'oxadiazolylméthylèneoxy qui est substitué par l'un des restes indiqués pour R¹, pour la préparation de compositions destinées à combattre des nématodes, des trématodes ou des cestodes.

6. Utilisation, suivant la revendication 1, de composés de formule (I), dans laquelle
R¹ représente de l'hydrogène, un groupe méthyle, éthyle, isopropyle ou sec.-butyle, trichlorométhyle ou trifluorométhyle,
R² est de l'hydrogène ou un groupe méthyle,
X représente O, S, SO, SO₂ et -N-R³,
R³ est de l'hydrogène, un groupe acétyle, propionyle ou trifluoracétyle,
R⁴ représente des restes identiques ou différents de la série hydrogène, fluor, chlore, brome, nitro, isothiocyanato, amino, méthylamino, éthylamino, diméthylamino, chlorure, bromure ou iodure de triméthylammonium, hydroxy, mercapto, méthyle, éthyle, isopropyle, butyle, hexyle, octyle ou nonyle, trifluorométhyle, méthoxy, éthoxy, méthylènedioxy ou éthylènedioxy, qui sont substitués, le cas échéant, par du fluor ou du chlore, trifluorométhoxy, fluorochloréthoxy, méthylthio, trifluorométhylthio, fluorochlorométhylthio, méthylsulfinyle, trifluorométhylsulfinyle, méthylsulfonyle, trifluorométhylsulfonyle ainsi que 3-(1,2,4-oxadiazolyl)-méthylènedioxy qui est substitué en position 5 par l'un des restes indiqués pour R¹,
pour la préparation de compositions destinées à combattre des nématodes, des trématodes ou des cestodes.

7. Composition contre les nématodes, les cestodes ou les trématodes, caractérisée par une teneur en au moins un dérivé substitué de 1,2,4-oxadiazole de formule (I) suivant la revendication 3.

8. Utilisation de dérivés substitués de 1,2,4-oxadiazole de formule (I) suivant la revendication 3 pour la préparation de compositions contre des nématodes, des cestodes ou des trématodes.
